# EUROPEAN PATENT APPLICATION

(11) **EP 2 868 348 A1**
(43) Date of publication of application: **06.05.2015**
(21) Application number: 13191456.6
(22) Date of filing: 04.11.2013
(51) Int. Cl.: A61P 9/00, A61P 11/00, A61P 13/12, A61P 1/00, A61K 31/28

(54) **CO-releasing compounds & formulations thereof useful for inducing mitochondrial biogenesis and tissue repair**

(71) Applicant: Universität Zürich, 8006 Zürich (CH); Duke University, Durham, NC 27705 (US)
(72) Inventor: Zobi, Fabio, 8052 Zürich (CH); Piantadosi, Claude, Durham, NC North Carolina 27705 (US); Suliman, Hagir, Durham, NC North Carolina 27705 (US)
(74) Representative: reuteler & cie SA

(57) **Abstract**

Provided are pharmacological methods of inducing through a unified set of molecular mechanisms the maturation of stem cells, methods of promoting mitochondrial biogenesis in a cell and tissue, methods of reducing pluripotency in a stem cell, and methods of promoting differentiation of a stem cell. In particular, the invention relates to methods for promoting tissue growth and/or tissue repair and/or improving tissue survival, preventing and treating fibrotic lesions and disorders

## Description

### FIELD OF THE INVENTION

The invention relates to the use of Carbon monoxide-releasing compounds (CORMs) based on electronically unsaturated Rhenium complexes to stimulate mitochondrial biogenesis, promote tissue repair, and combat fibrosis.

### BACKGROUND OF THE INVENTION

Carbon monoxide (CO) is both a toxic gas and a physiological molecule. Its toxicity is caused by chemical asphyxia through tight binding to the hemoglobin molecule, forming carboxyhemoglobin (COHb), which does not carry oxygen, and through binding to intracellular heme proteins such as myoglobin and cytochrome c oxidase, which are involved in oxygen transport and utilization by cells for the production of ATP. CO is produced naturally in the body by the heme oxygenase enzymes (HO-1 and HO-2), which catalytically degrade heme to produce biliverdin, releasing iron and CO in the process. CO is excreted by the lungs and the normal COHb level is 1-2% in the bloodstream.

During certain disease states, HO-1 is induced and COHb levels can rise to 3-6%. The CO released in these conditions has physiological effects that promote cell survival. These effects include protection against certain types of inflammatory and oxidative damage and cell death by apoptosis. Due to the highly demanding dose control of inhaled CO, to its high toxicity and lack of specificity, to safety considerations for its administration in hospital settings, and also to due to the lack of patients' compliance to CO inhalation, there has been considerable interest in the development of CO-releasing molecules or "CORMs" as an alternative to the administration of CO gas.

CORMs do not target a specific receptor but their potential utility is generally ascribed to their fundamental ability to liberate CO. CORMs are therefore used as systemic prodrugs to deliver CO as active ingredient. Most CORMs are molecules tailored around a transition metal ion, mainly metallo-carbonyls or borano carbonates, which have their own set of toxicities and lack tissue or cell targeting properties, and present issues in production homogeneity and/or water solubility and/or biocompatibility and/or stability and/or metabolites identity. Further, those CORMS still proved to have adverse effects on mitochondrial energetics and integrity *(*Winburn et al., 2012, Basic Clin. Pharmacol. Toxicol., 111, 31-41*).*

Rhenium-based CORMS have been found to be useful in the treatment of cardiovascular diseases, ischemia-reperfusion injury, inflammatory diseases, traumatic injury and xenograft rejection, neuron degeneration of the nervous system, radiation damage, cancer, penile erectile dysfunction, adult respiratory distress syndrome, and disorders of the circadian rhythm of mammals (WO 2011/110315).

Fibrosis is a fundamental constituent of cardiac, pulmonary, hepatic, as well as renal pathologies and occurs as a severe co-morbidity in several diseases and various types of organs injuries. It involves a deregulated cross talk between epithelial cells and the adjacent layer of fibroblasts or mesenchymal cells (the epithelial-mesenchymal-unit), causing (i) hyperplasia of fibroblast-like mesenchymal cells, (ii) an exaggerated production and abnormal accumulation of extracellular matrix proteins as well as changes in its composition, including deposition of collagen and fibronectin, degradation of certain enzymes (such as matrix-metalloproteinase (MMP) and their inhibitors TIMPs (tissue inhibitors of metalloproteinases), or changes in the level of post translational modifications of specific extracellular matrix components, (iii) deregulation of epithelial to mesenchymal transition. Reduced mitochondrial biogenesis is common to multiple fibrotic lesions. Several animal models of heart disorders involving myocardial fibrosis, such as obesity-linked heart failure *(*De Pergola et al., 2013, Endocr. Metab. Immune Disord. Drug Targets*),* cardiac steotasis *(*Glenn et al., 2011, Hypertension, 57, 216-22*),* or age-dependent cardiomyopathy *(*Dai et al., 2010, Aging Cell, 9, 536-44*)* also exhibit reduced mitochondrial biogenesis. Therapeutic approaches to fibrosis that lead to up-regulation of mitochondrial biogenesis, such as mitochondrial-targeted antioxidant peptide SS-31 *(*Dai et al., 2011, J. Am. Coll. Cardiol., 58, 73-82*)* or Eyes absent 2 expression *(*Lee et al., 2009, J. Mol. Cell. Cardiol., 46, 596-605*),* indeed ameliorate cardiac dysfunction and prevent fibrosis in hypertensive cardiomyopathy models and cardiac hypertrophy models, respectively. Similarly, results with peptides providing some protection against renal fibrotic damage in a model of atherosclerotic renal artery stenosis and a unilateral urethral obstruction model of renal function were shown to effectively stimulate mitochondrial biogenesis *(*Eirin et al., 2012, Hypertension, 60, 1242-9*);* Mizuguchi et al., 2008, Am. J. Physiol. Renal. Physiol. 295, F1545-53*).* In addition, it was recently shown that the anti-fibrotic and anti-apoptotic effects of a vitamin D receptor activator in a mouse obstructive nephropathy model involved protection against mitochondrial injury *(*Garcia et al., 2012, Am. J. Physiol. Renal Physiol., 302, F1595-605*).* A correlation between renal fibrosis and disrupted renal mitochondrial biogenesis has also been evident in aged kidneys or in models of diabetic renal injury *(*Melk et al., 2005, Kidney Int., 68, 2667-79*;* Hwang et al., 2012, Diabetes, 61, 728-38*).* In the lung, transforming growth factor beta (TGF-β1), which mediates lung fibrosis, was shown to decrease key activators of mitochondrial biogenesis *(*Sohn et al., 2012, Cell. Mol. Biol. (Noisy-le-grand), Suppl.58, OL1763-7*).* For the liver, disruption of mitochondrial biogenesis participates in exacerbating liver injury fibrosis *(*Song et al., 2011, J. Pharmacol. Exp. Ther., 339, 298-306*).*

Therefore, there is a need to for providing methods and substances which would be useful in stimulating mitochondrial biogenesis and/or preventing or reducing the development of fibrotic lesions or fibrosis in organs, notably in cardiac, pulmonary, hepatic, as well as renal pathologies, and thereby improve organ function and patient survival.

### SUMMARY OF THE INVENTION

The present invention relates to the unexpected finding that inducing expression of heme oxygenase 1 (HO-1) in a cell, which activates mitochondrial biogenesis, thereby improves the resistance of tissues to multiple forms of stress by stimulating stem cell maturation, reducing apoptosis, and preventing fibrosis. The present invention further relates to the unexpected finding that a family of CORMs, Re-based CORMs and derivatives, is able to specifically induce the expression of heme oxygenase 1 (HO-1) in a cell and genetically stimulate mitochondrial biogenesis, through which a network of anti-inflammatory, anti-fibrotic, and tissue repair effects are genetically activated to prevent further and/or reverse existing or ongoing damage and cell death in tissues and organs. The present invention further relates to the unexpected finding that a family of CORMs, Re-based CORMs, is able to specifically stimulate stem cell differentiation, and thereby block or prevent fibrosis in a broad range of tissues and organs and diseases. In an aspect, the invention provides a method of specifically inducing mitochondrial biogenesis and eliciting pro-survival effects in a cell, a tissue or an organ, said method comprising contacting said cell, tissue or organ with a Re-based CORM, a derivative thereof of a mixture thereof or administering a Re-based CORM, a derivative thereof or a mixture thereof to said cell, tissue or organ.

According to one aspect, the invention provides a method of preparation of a cell composition for cell-based therapy comprising a step of inducing expression of heme oxygenase 1 (HO-1) in a stem, or a progenitor, or a precursor, or a totipotent, or a pluripotent, or a multipotent or an oligopotent, or a bipotent, or an unipotent cell.

In another aspect, the invention provides a method of preparation of a cell composition for cell-based therapy comprising a step of contacting cells, in particular stem cells, with a Re-based CORM, a derivative thereof or a mixture thereof.

In another aspect, the invention provides a method for treating or preventing fibrosis or fibrotic lesions.

In another aspect, the invention provides a method for promoting tissue growth and/or repair, in particular for improving cell/tissue survival.

In another aspect, the invention provides Re-based CORMs and derivatives or a mixture thereof for use in the treatment or prevention fibrosis or fibrotic lesions.

In another aspect, the invention provides Re-based CORMs and derivatives or a mixture thereof for use in the promotion of tissue growth and/or repair and/or improving cell/tissue survival.

In another aspect, the invention provides Re-based CORMs and derivatives or a mixture thereof for use in the inducing mitochondrial biogenesis and eliciting pro-survival effects in cells, tissues and organs, in particular for use in the inducing or promoting mobilization, differentiation, maturation, proliferation, and survival of stem cells.

In another aspect, the invention provides a use of Re-based CORMs, derivatives thereof or a mixture thereof for the preparation of a cell preparation or a pharmaceutical composition for inducing mitochondrial biogenesis and eliciting pro-survival effects in cells, tissues and organs.

In another aspect, the invention provides a use of Re-based CORMs, derivatives thereof or a mixture thereof for the preparation of a cell preparation or a pharmaceutical composition for promoting tissue growth and/or repair and/or improving cell/tissue survival, in particular for treating or preventing fibrosis or fibrotic lesions.

In another aspect, the invention provides a preservation or preparation medium for cells, organs or tissues comprising a Re-based CORM, a derivative thereof or a mixture thereof.

In another aspect, the invention provides a composition for preservation or preparation of cells, organs or tissues or a pharmaceutical composition comprising Re-based CORMs, derivatives thereof or a mixture thereof combined with at least one co-agent, in particular compositions which are useful in the promotion of tissue growth, repair or survival and/or useful in the improvement of cell survival, and/or useful in the treatment or prevention of fibrosis or fibrotic lesions.

In another aspect, a cell preparation comprising Re-based CORMs, derivatives thereof or a mixture thereof combined with at least one co-agent useful in the preparation of stem cells.

In another aspect, the invention provides a method of promoting mitochondrial biogenesis in a cell comprising inducing expression of HO-1 in said cell.

According to a particular aspect, methods, compositions and uses of the invention may comprise inducing expression of HO-1 in the cell without causing or inducing damage to existing or intact mitochondria in the cells.

The disclosure provides for other aspects and embodiments that will be apparent in light of the following detailed description and accompanying Figures.

### BRIEF DESCRIPTION OF THE DRAWINGS

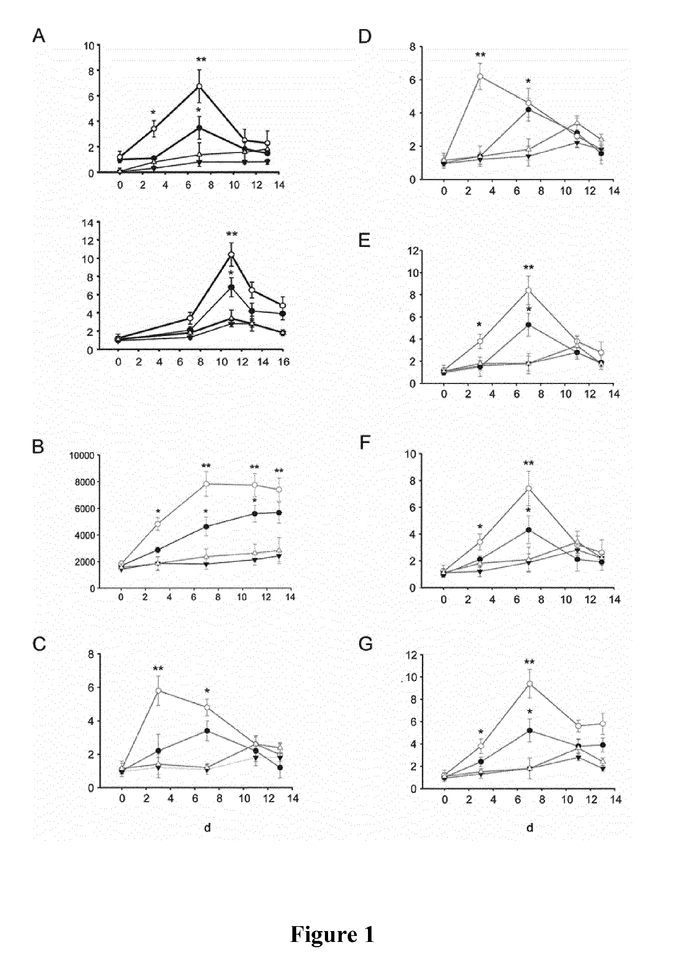

**Figure 1** shows the effect of B12-CORM2 on the regulation of mitochondrial biogenesis in E14 stem cells (ESC) during differentiation into cardiomyocytes as described in Example 1. E14 cells were not treated (black circles) or treated with B12-CORM2 (25 µM) (open circles) or submitted to HO-1 gene silencing (Sh-HO-1) (black triangles) or treated with both B12-CORM2 and Sh-HO-1 (open triangles). mRNA expression levels were assessed for up to 13 days in culture (d). **(A)** HO-1 mRNA expression (above panel) during differentiation with or without B12-CORM2 treatment or HO-1 gene silencing (Sh-HO-1) or both (top panel A). Also shown is citrate synthase (CS) (below panel), a TCA cycle enzyme that indicates change in mitochondrial mass (bottom panel A). **(B)** MtDNA copy number during spontaneous and B12-CORM2-induced differentiation. MtDNA was quantified by RT-PCR and normalized to the 18S gene. **(C-F)** Expression of NRF-1 (C), PGC-1α (D), Poly (E), and Tfam (F) transcriptional regulators of mitochondrial biogenesis quantified by real-time RT-PCR. Expression of each gene was normalized to 18S and expressed as fold change in gene expression with respect to the undifferentiated E14 ESC (at day 0). **(G)** Expression of mitochondrial gene COII mRNA determined using real-time RT-PCR showing functionality of mtDNA. Data are presented as fold change in gene expression with respect to day 0. The values represent mean ± SEM of three independent determinations. Significant differences between control, B12-CORM2 treated, sh-HO1 transfected, and sh-HO1 transfected and treated with B12-CORM2 are indicated *, P<0.05; **, P<0.01. Three independent experiments were performed.

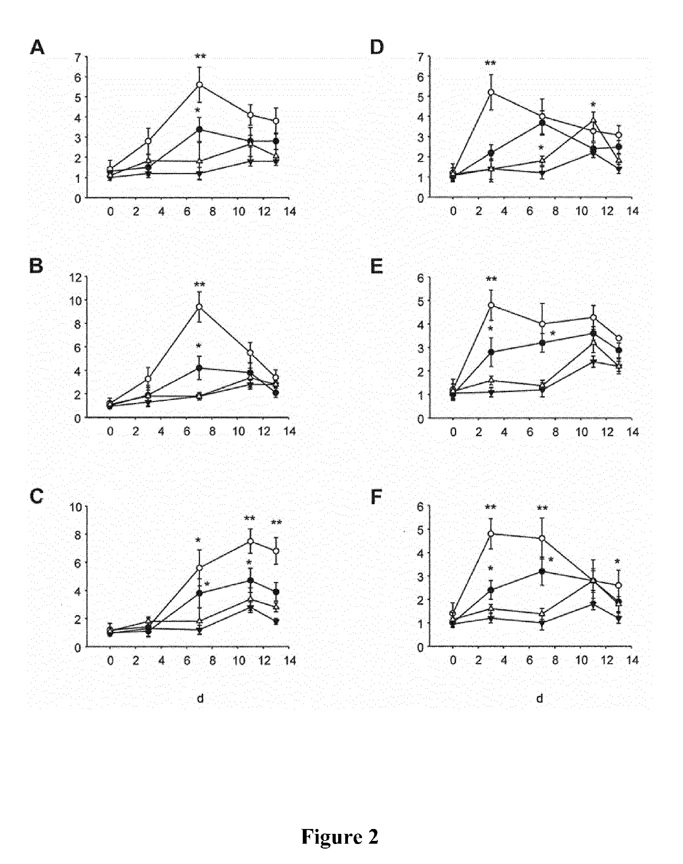

**Figure 2** shows the effect of B12-CORM2 on induction of myocardial differentiation markers as presented in Example 1. E14 cells were not treated (black circles) or treated with B12-CORM2 (25 µM) (open circles) or submitted to HO-1 gene silencing (Sh-HO-1) (black triangles) or treated with both B12-CORM2 and Sh-HO-1 (open triangles). mRNA expression levels were assessed for up to 13 days in culture (d). **(A-C)** Assessment of cardiac structural maturity markers **(A)** MLC2a, **(B)** troponin I (cTnl), and **(C)** atrial natriuretic peptide (ANP) mRNA expression. **(D-F)** Demonstration of B12-CORM2 or HO-1 gene silencing effects on the expression of major transcription factor genes that drive structural transformation of ESC into cardiomyocytes **(D)** Gata4, **EB)** Nkx2.5, and **(F)** Mef2c mRNA expression during ESC differentiation. Values represent mean ± SEM. Significant differences between control, B12-CORM2 treated, sh-HO1 transfected, and sh-HO1 transfected and treated with B12-CORM2 are indicated *, P<0.05; **, P<0.01. Three independent experiments were performed. In each case, the ability of B12-CORM2 to effect differentiation depends on the presence of HO-1. The mRNA levels for the indicated genes were analyzed by real-time RT-PCR and normalized to the 18S gene and presented as fold expression change in mRNA with respect to day 0. Day 0 represents undifferentiated cell culture collected before subjecting the cells to differentiation media and B12-CORM2.

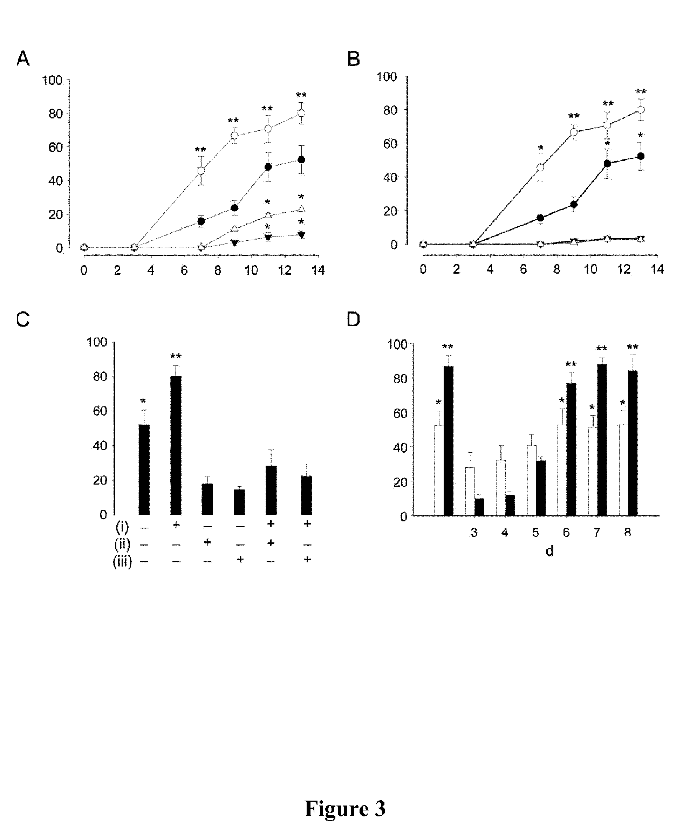

**Figure 3** shows the differentiation of embryoid bodies (EB) into beating cardiomyocytes under the control of HO-1 and B12-CORM2 as presented in Example **1. (A)** and **(B)** show the percentage of EBs that contain beating areas at days (d) 7 to 13. Black circles indicate untreated EBs, open circles EBs exposed to B12-CORM2 (25 µM), and black triangles EBs submitted to HO-1 gene silencing (Sh-HO-1) in **(A)** or cells unable to express Tfam in **(B).** Open triangles represent cells treated with B12-CORM2 and Sh-HO-1 in **(A)** or cells treated with B12-CORM2 and unable to express Tfam in **(B).** Results are expressed as a percentage of the number of EBs with beating foci with respect to the total number. Values represent mean ± SEM. Significant differences between control, B12-CORM2 treated, shHO-1 transfected, and shHO-1 transfected and treated with B12-CORM2 are indicated *, P<0.05; **, P<0.01. Three independent experiments were performed. (C) Percentage of EBs that contain beating areas following treatment with B12-CORM2 (i) and/or insertion of mitochondrial-targeted catalase (mCAT) (ii) and/or treatment with the mitochondrial antioxidant, MitoQ (iii), that block B12-CORM2-induced cardiac differentiation. **(D)** Percentage of EBs that contain beating areas following B12-CORM2 treatment (black) or not (white) and MitoQ given in the initial five days (d) of differentiation. MitoQ blocks the B12-CORM2 effect on the percentage of beating EB.

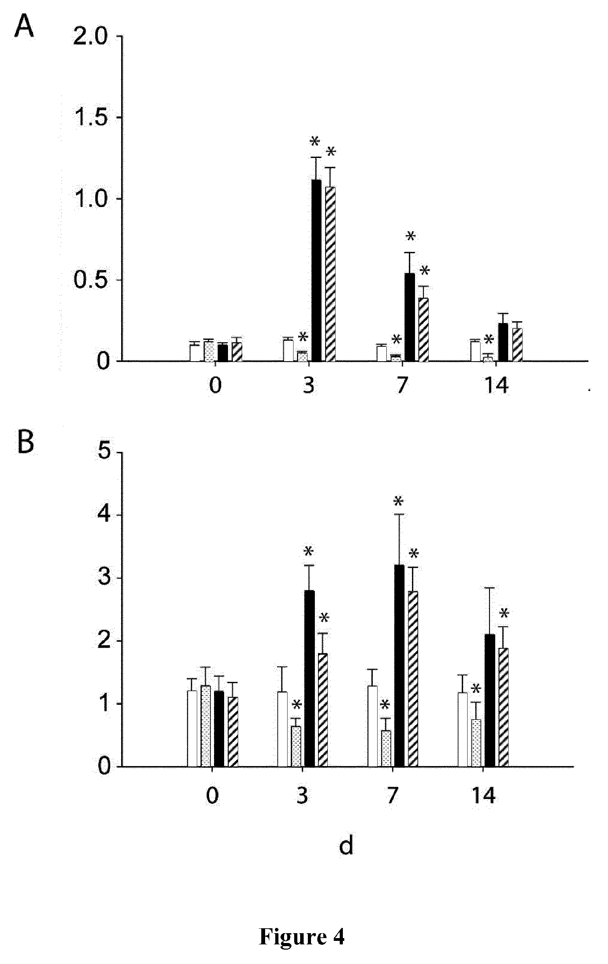

**Figure 4** demonstrates improved cardiac repair of DOX-injured mouse heart *in vivo* using B12-CORM2 therapy for 3 days at the time of DOX administration. White columns indicate untreated control mice. Dotted columns indicate mice exposed to DOX (15 mg/kg). B12-CORM2 (30 mg/kg) was administered subcutaneously once daily for three days in control mice (black columns) or mice exposed to DOX (striped columns). mRNA levels of cardiac aurora kinase and cardiac Nkx2.5 were analyzed by real-time RT-PCR at days 0, 3, 7, and 14 (d). **(A)** shows the capacity of B12-CORM2 to increase, after DOX administration, cardiac aurora kinase mRNA levels, presented here as fold expression change in mRNA with respect to day 0. **(B)** shows the capacity of B12-CORM2 to increase, after DOX administration, the expression of cardiac Nkx2.5, presented here as fold expression change in mRNA with respect to day 0. (*, P<0.05 vs. day 0).

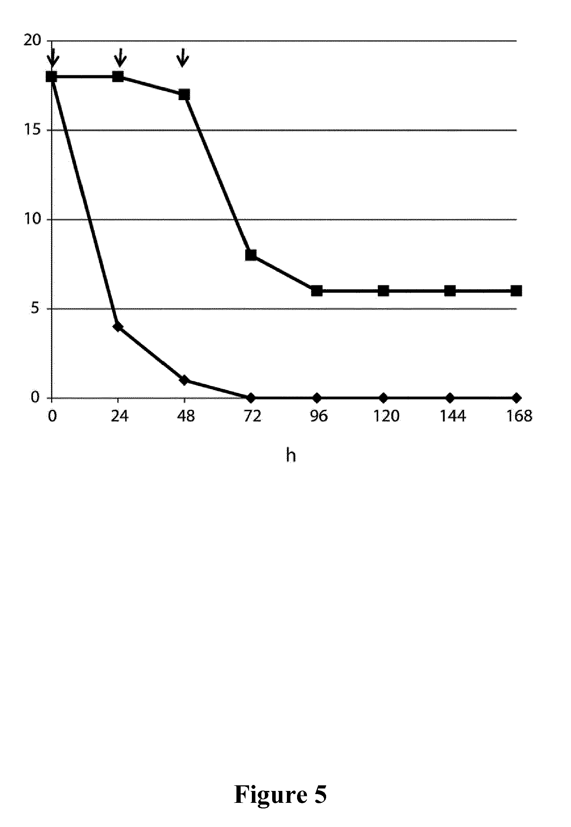

**Figure 5** presents the number of surviving mice inoculated with live *S. aureus* and treated with B12-CORM2 (squares) or not treated (diamonds). Mice were inoculated with live *S. aureus* at a dose of 10⁸ CFU, treated with Vancomycin (3 mg/kg), and followed for 7 days (168 hours (h)). Arrows indicate subcutaneous injections of 30 mg/kg B12-CORM2 once-daily for 3 days in 5 %DMSO (n=18) or subcutaneous injections of control 5% DMSO solution (vehicle) (n=18). B12-CORM2-treated mice exhibit a significant survival advantage in live S. *aureus* sepsis as shown by significantly decreased two- and seven-day mortality (P<0.05).

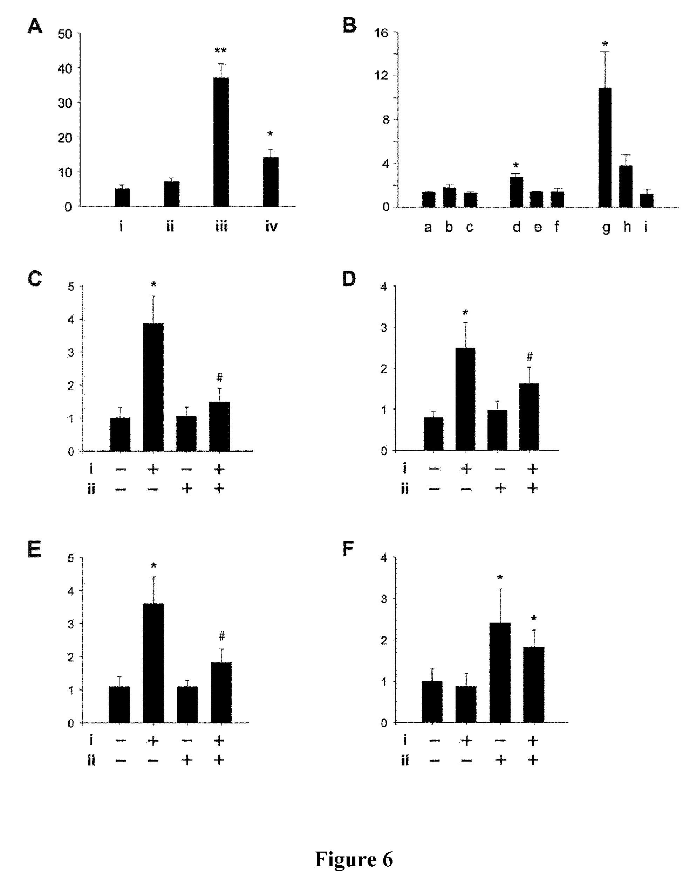

**Figure 6** shows the protective effects of B12-CORM2 in lung cells against cytomix-induced cell death and against pro-fibrotic states. **(A)** Percentage of lung alveolar type II (MLE12) cell death after treatment with B12-CORM2 (ii), or after exposure to an LD50 of LPS, TNF-α, and IL-1β (cytomix) (iii), or after exposure to both cytomix and B12-CORM2 (iv), demonstrating the pro-survival effects of B12-CORM2. (i) corresponds to untreated cells. Three to four independent experiments were performed. (*, P<0.05 vs. control; **, P<0.05 vs. cytomix alone). **(B)** TGFβ1-induced production of collagen-1a (mRNA expression levels) in human lung MRC5 fibroblasts at 0 (a, b, c), 2 (d, e, f), and 6 (g, h, i) days in culture. (a, d, g) indicate TGF-β-treated cells (5 ng/ml). (b, e, h) indicate B12-CORM-2-treated cells (25 µM) exposed to TGF-β (5 ng/ml). (c, f, i) indicate cells treated with B12-CORM2 only (25 µM). (*, P<0.05 vs. control; #, P<0.05 vs. control and TGF-β). Pro-fibrotic state induced by the chemotherapeutic drug Bleomycin (100 milliunits/ml media) (i) in human lung MRC5 fibroblast cells, as revealed by increased collagen-1a mRNA expression. A single B12-CORM2 (ii) (25 µM) treatment blocks collagen-1a mRNA expression (C) and inhibits activation of TGF-β1 **(D)** and Smad3 **(E). (F)** B12-CORM2 simultaneously increases NRF-1 mRNA levels in the presence of Bleomycin. (*, P<0.05 vs. control; #, P<0.05 vs. control and Bleomycin).

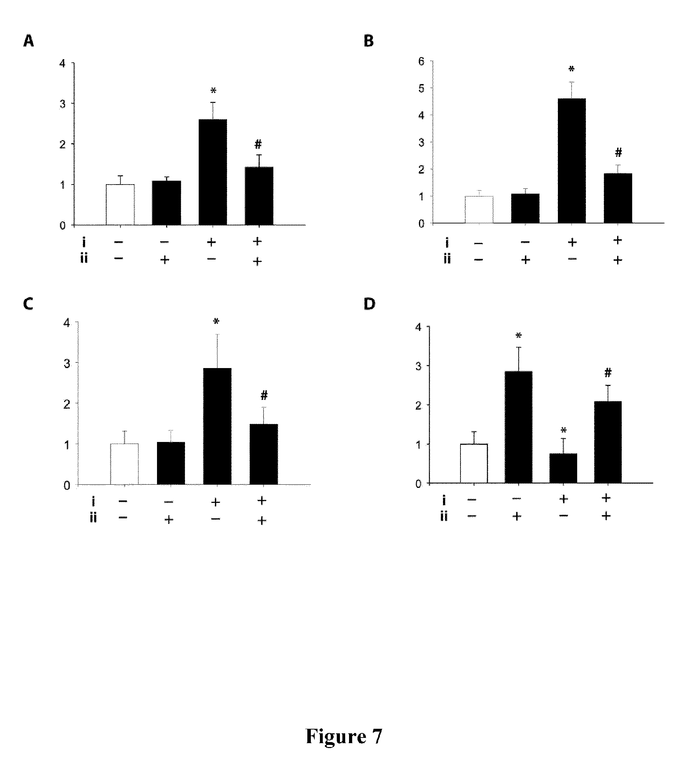

Figure 7 shows B12-CORM2 blocking effects in a mouse Bleomycin-induced lung fibrosis model. Bleomycin (i) was injected into the mouse lung (1 unit/kg subcutaneously for 3 days) and the lungs were harvested 7 days later for analysis. Bleomycin administration increases pulmonary gene expression (fold change) for **(A)** TGF-β1, **(B)** Smad3, **(C)** fibronectin, and **(D)** decreases mitochondrial volume density in the lung parenchyma. The administration of B12-CORM2 (ii) blocks the increases in **(A)** TGF-β1, **(B)** Smad3, **(C)** fibronectin, and **(D)** protects mitochondrial volume density (fold change). (*, P<0.05 vs. control; #, P<0.05 vs. Bleomycin).

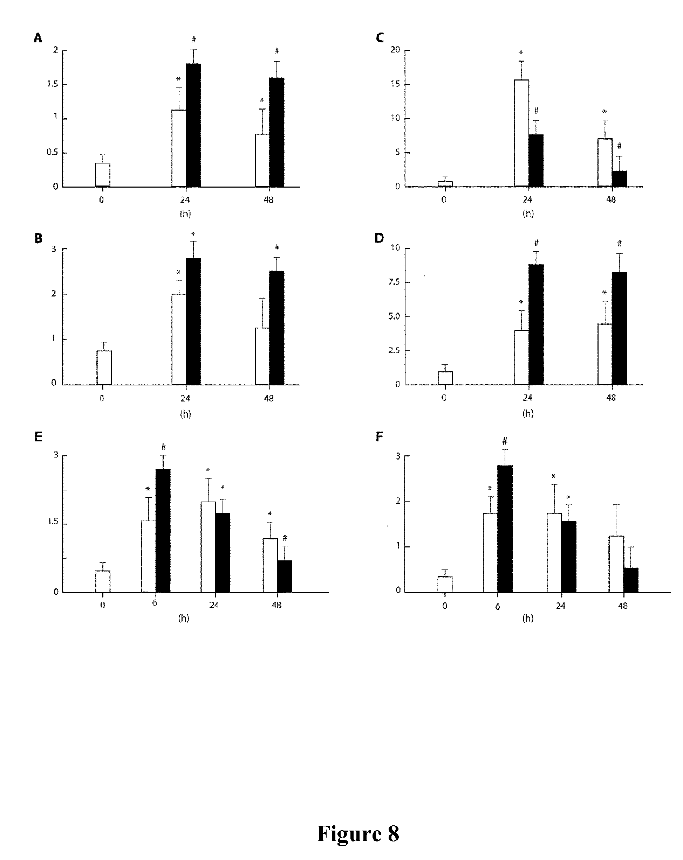

**Figure 8** demonstrates the pro-survival and anti-inflammatory effects of B12-CORM2 as well as its impact on the induction of mitochondrial autophagy (mitophagy) in mouse lung following *S. aureus* sepsis. WT mice underwent fibrin-clot model with Vancomycin (3 mg/kg) plus fluid resuscitation (1.0 ml subcutaneously) (white) or the same inoculation plus three injections of B12-CORM2 (30 mg/kg/day subcutaneously) (black). 24 and 48 hours (h) following *S. aureus* sepsis, lungs were harvested and protein extracted for **(A)** pAkt/Akt ratio, (B) HO-1 protein by Western analysis and **(C)** TNF-α and **(D)** IL-10 levels by ELISA. The Western blots were analyzed by optical densitometry. The B12-CORM2 treated mice showed more robust and prolonged pro-survival pAkt and HO-1 induction and lower TNF-α and higher anti-inflammatory IL-10 levels than vehicle-control mice. **(E-F)** Activation of mitophagy in the lung was assessed during sepsis with and without B12-CORM2 treatment. During infection, B12-CORM2 accelerated the induction and allowed the earlier resolution of autophagy in the lung as shown by changes **(E)** p62 and **(F)** LC3B marker protein levels at 6, 24 and 48 hours (h) following S. *aureus* sepsis, compared with untreated mice. (n=4 per group; * P<0.05 compared with control; # P<0.05 compared with sepsis only).

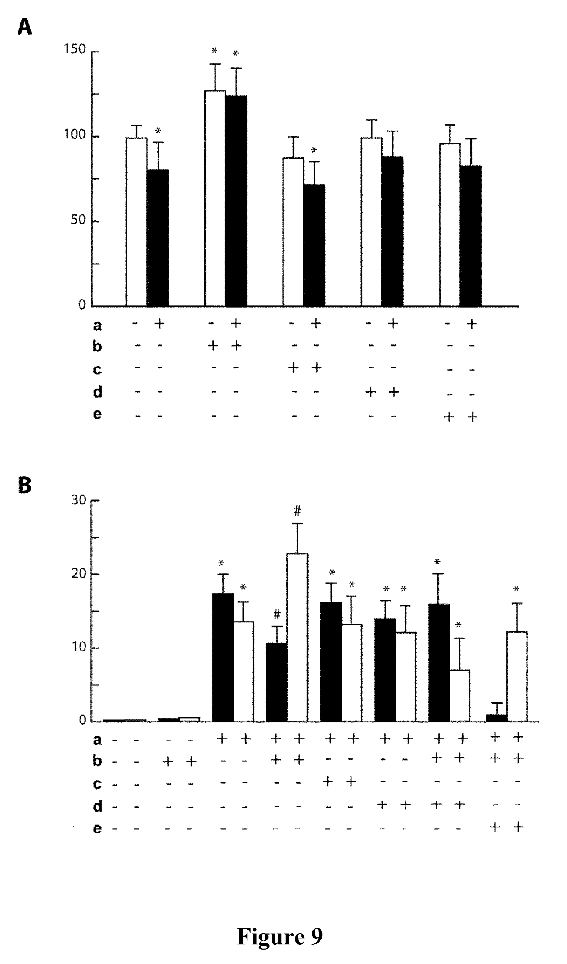

**Figure 9** shows that Hepa 1-6 cells display enhanced metabolic capacity, resistance to cytomix-induced loss of metabolic capacity and reduction in inflammatory response after B12-CORM2 treatment. Mouse hepatoma cells (ATCC, CRL-1830) were grown to near confluence according to the manufacturer's instructions and then exposed to cytomix (a) for 24 hours. At the time of cytomix exposure, cells were treated with B12-CORM2 (25 µM) (b) or the other compounds shown in panel (A). **(A)** The MTT assay, data shown as MTT Abs (% control), demonstrates the pro-metabolic effects and lack of cytotoxicity of the drug as compared to the HO-1 substrates, hemin and bilirubin. **(B)** shown the relative mRNA levels for the pro-inflammatory enzyme NOS2 (black) and the anti-inflammatory cytokine interleukin 10 (IL10) (white), as measured by real time RT-PCR. Control and cytomix-treated cells were harvested, disrupted, and total RNA extracted. Both gene products increased in response to cytomix, but B12-CORM2 reduced NOS2 and enhanced IL10 expression. The requirement for CO in the effect of a Re-based CORM according to the invention is demonstrated by positive and negative control data using the HO-1 substrate hemin, the HO-1 product bilirubin, and inactive controls (B12-ReAA and NaReO4) (c). Moreover, administration of the low molecular weight anti-oxidant N-acetyl cysteine (NAC, 5 mM) (d) abrogates the anti-inflammatory effect of the Re-based CORM according to the invention in accordance with the proposed redox mechanism of action. Finally, the NF-kB inhibitor BAY11 (BAY 11-7082, Santa Cruz) (e) fully blocks the cytomix effect on NOS2, but only partially reduces the effect on IL-10, indicating the NF-kB-independent mechanism of action of B12-CORM2 (*, P<0.05 compared with control cells; #, P<0.05 compared with cytomix treatment).

### DETAILED DESCRIPTION

Aspects of the methods, uses and compositions disclosed herein are based on the novel and unexpected properties of Re-based CORMs which are found to be able to stimulate stem cell differentiation and prevent fibrosis. In particular, Re-based CORMs are found to be able to stimulate mitochondrial biosynthesis through a retrograde mechanism that promotes the translocation of HO-1 to mitochondria for degradation of heme in damaged mitochondria and the replacement of such mitochondria with healthy mitochondria by recycling components through autophagy and mitochondrial biosynthesis. Those properties of Re-based CORMs can be advantageously used to produce a novel, rapid, and specific induction of genes related to the terminal differentiation of stem cells, while protecting the cells from a pro-fibrotic phenotype and preventing fibrosis development. In particular, Re-based CORMs and derivatives may be utilized to stimulate cell regeneration and to prevent and/or treat tissue fibrosis.

The term "tissue" includes heart tissue, renal tissue, liver tissue, and lung tissue, including type II epithelial cells.

The terms "preservation agent" or "preservation compositions" include agents or compositions used to effectively, safely, and reliably improve organs or tissues or cell storage, maximize organ function after reperfusion, and improve outcomes after transplantation. Since preservation methods aim at inducing hypothermia to reduce the metabolic requirements of the organ, preservation solutions have been developed to further extend acceptable cold ischemia times and to counteract the deleterious adverse effects associated with hypothermia (cold ischemia) and with reperfusion on post-transplant organ function. Although the composition of preservation compositions listed herein may vary, their purpose all aim at preventing cellular edema, counteracting acidosis, delaying cell destruction, scavenging and reducing the evolution of free radicals, minimizing membrane pump activity, and maintaining cellular ATP levels. Organ preservation compositions may consist of cold preservation compositions, which are used for cold hypothermic static preservation, a standard method for the preservation of perfused organs/tissues at approximately 4°C. Organ preservation compositions may also consist in compositions for warm or normothermic preservation, which is used to overcome some limitations of cold storage (such as the limitation of ischemic preservation, vascular endothelial damage, absence functional improvement) and to allow the assessment of organ function ex *vivo* and improve immunological compatibility. Warm preservation compositions may also consist of nutrients added to blood.

The term "fibrosis" or "fibrotic lesions" includes the pathological formation of fibrous tissue due to the hyperplasia of fibroblast like mesenchymal cells, to the exaggerated production, abnormal accumulation, and composition modification of extracellular matrix proteins, and to the deregulation of epithelial to mesenchymal transition. "Fibrosis" or "fibrotic lesions" characterized in part by extensive collagen deposition may be a constituent and a consequence of cardiac, pulmonary, renal, hepatic, or systemic pathologies.

The term "cell" according to the invention refers to stem cells, progenitor or precursor or totipotent or pluripotent or multipotent or oligopotent or bipotent or unipotent cells. Cells may further refer to embryonic stem cells (ESC) or post-natal or adult stem cells. Stem cells may further include cardiac, hepatic, renal, pulmonary, hematopoietic, or neuronal progenitor cells. According to a particular aspect, those cells are allogeneic and autologous.

The term "stem cells" refers to biological cells, found in all multicellular organisms, that can divide through mitosis and differentiate into diverse specialized cell types and can self-renew to produce more stem cells. In mammals, there are two broad types of stem cells: embryonic stem cells, which are isolated from the inner cell mass of blastocysts (early-stage embryos), and adult stem cells, which are found in various tissues. In adult organisms, stem cells can divide or self-renew indefinitely, and generate all the cell types of the organ from which they originate, potentially regenerating an entire organ from a few cells. Adult stem cells are "multipotent" cells. In a developing embryo, stem cells can differentiate into all the specialized cells ("pluripotent" cells), but also maintain the normal turnover of regenerative organs, such as blood, skin, or intestinal tissues.

As used herein, the term "progenitor cell" or "oligopotent cell" refers to a multipotent cell that, like a stem cell, has a tendency to differentiate into a specific type of cell, but is already more specific than a stem cell and is pushed to differentiate into its "target" cell. Another difference between stem cells and progenitor cells is that stem cells can replicate indefinitely, whereas progenitor cells can divide only a limited number of times.

As used herein, the term "precursor cell" refers to a stem cell which has lost most of its multipotency to become a unipotent, partially-differentiated, cell. A "unipotent cell" used herein refers to a stem cell that will only differentiate into one cell type.

As used herein, the term "bipotent cell" refers to a stem cell which may differentiate into one or another cell type.

The "potency" of a cell specifies its differentiation potential, or potential to differentiate into different cell types. "Totipotency" is the ability of a single cell to divide and produce all the differentiated cells in an organism, including extra embryonic tissues. Totipotent cells include spores and zygotes. "Pluripotency" refers to a stem cell that has the potential to differentiate into any of the three germ layers: endoderm (interior stomach lining, gastrointestinal tract, the lungs), mesoderm (muscle, bone, blood, urogenital), or ectoderm (epidermal tissues and nervous system). Pluripotent stem cells can give rise to any fetal or adult cell type. However, alone they cannot develop into a fetal or adult organism because they lack the potential to contribute to extra embryonic tissue, such as the placenta. "Multipotent" progenitor cells have the potential to give rise to cells from multiple, but a limited number of lineages. An example of a multipotent stem cell is a hematopoietic cell, a blood stem cell that can develop into several types of blood cells, but cannot develop into brain cells or other types of cells.

The term "Induced pluripotent stem cells" ("iPS") are adult cells that have been genetically reprogrammed to an embryonic stem cell-like state by being forced to express genes and factors important for maintaining the defining properties of embryonic stem cells. Mouse iPS cells were first prepared in 2006 *(*Takahashi and Yamanaka, Cell, 2006; 126: 663-676). Human iPS cells were prepared in 2007 *(*Takahashi et al, Cell, 2007, 131: 861-872*;* Yu et al, Science, 2007; 318: 1917-1920*).* These human iPS cells are similar to human embryonic stem cells in their morphology, their gene expression, and the epigenetic status of pluripotent cell-specific genes. Their therapeutic potential is examined for multiple clinical applications, for example iPS-derived cardiomyocytes have been developed for heart repair, iPS cells are used for engineering lung epithelia tissue, iPSC-derived hepatocyte-like cells have been generated for liver diseases, and iPS cell lines have been produced from adult kidney-derived cells *(*Dierickx et al., 2012, J. Cardiovasc. Transl. Res, 5(5), 566-80*;* Kotton, 2012, Am. J. Respir. Crit. Care Med., 185(12), 1255-60*;* Lau et al., 2012, Mol. Ther., 20(6), 1116-30*;* Wetsel et al., 2011, Annu. Rev. Med., 62, 95-105*;* Yi et al., 2012, Protein Cell., 3(4), 246-50*;* Hendry and Little, 2012, Kidney Int., 82(2), 138-46).

The term "cell-based therapy" or "cell-based tissue regeneration" include cell replacement therapies making use of allogenic or autologous stem cells, or in the direct induction of tissue regeneration by *in situ* stimulation of resident stem cells (e.g. inducing resident stem cells mobilization and differentiation for repair), as alternatives to surgical interventions and organ/tissue transplantation. Methods and compositions according to invention can be advantageously used in methods of "cell-based therapy" or "cell-based tissue regeneration" methods used to produce differentiated tissue from progenitor cells or stem cells such as stem cells of cardiac, pulmonary, hepatic, renal or bone marrow origin as listed herein.

The term "Non-infectious systemic inflammatory response syndrome (SIRS)" or "Systemic inflammatory response syndrome (SIRS) without infection" as used herein refers to SIRS associated with an established underlying non-infectious diagnosis and no reason to suspect any on- going infection. SIRS is commonly defined as the presence of two or more of the following parameters: body temperature greater than 38°C or less than 36 °C; heart rate greater than 90 beats per minute; respiratory rate greater than 20 breaths per minute; Pe02 less than 32 mm Hg; and a white blood cell count either less than 4.0 x 109 cells/L or greater than 12.0 x 109 cells/L, or having greater than 10% immature band forms. SIRS may be self-limited or lead to a multiple organ dysfunction syndrome (e.g., varying degrees of fever, hypoxemia, tachypnea, tachycardia, endothelial inflammation, myocardial insufficiency, hypoperfusion, altered mental status, vascular collapse, which may culminate in end-organ damage such as acute respiratory distress syndrome, coagulopathy, cardiac failure, renal failure, shock, and/or coma) (see: American College of Chest Physicians/Society of critical Care Medicine Consensus Conference, crit. Care Med., 1992; 20:864; and Bone RC, JAMA, 1995; 273:155).

The term "Sepsis" is a potentially serious medical condition that is characterized by a whole-body inflammatory state (called a systemic inflammatory response syndrome or SIRS) and the presence of either a proven (on the basis of sampling or radiology) or probable (considering the patient's clinical presentation, white cell count, CRP, radiology) infection. The term sepsis summarizes clinical pictures, for which, as a rule, fever, leucocytosis, consciousness changes, a hyperdynamic circulation ("warm shock"), and a hyper-metabolic status, mainly as a consequence of the invasion of the normally sterile tissue by microorganisms, are observed. The term sepsis corresponds to the definition of sepsis as defined in the "Definitions for Sepsis and Organ Failure and Guidelines for the Use of Innovative Therapies in Sepsis" from the ACCP/SCCM consensus conference committee (Bone et al., Chest 1992, 101;1644-1655) According to this definition, sepsis corresponds to a severe inflammatory response syndrome caused by the presence of an infection (either proven or probable). The infection may be cause by bacteria, virus or fungi. Triggers of sepsis include pneumonia, such as ventilator-associated pneumonia, abdominal infection, kidney infection, and bloodstream infection. The body may develop this inflammatory response by the immune system to microbes in the blood, urine, lungs, skin, or other tissues. A lay term for sepsis is blood poisoning, also used to describe septicaemia. Septicaemia is a related medical term referring to the presence of pathogenic organisms in the bloodstream, leading to sepsis. The modern concept of sepsis is that the host's immune response to the infection causes most of the symptoms of sepsis, resulting in hemodynamic consequences and damage to organs. This immunological response causes widespread activation of acute-phase proteins, affecting the complement system and the coagulation pathways, which then cause damage to the vasculature as well as to the organs. Various neuroendocrine counter- regulatory systems are then activated as well, often compounding the problem. Even with immediate and aggressive treatment, this may progress to multiple organ dysfunction syndrome and eventually death.

According to a particular aspect, methods and compositions according to invention are useful for the promotion of cardiac repair, in particular for replacing lost tissues in the damaged heart as an alternative to cardiac surgery or heart transplantation. The heart possesses a limited regeneration or repair capacity, and cardiac repair may occur in response to stresses that damage the heart. Moreover, this repair may also be stimulated *in situ* according to the invention. A niche of resident cells with multipotent cardiac stem cell characteristics has been identified in the post-natal and adult heart, and suggested to maintain cardiac integrity and to participate in the response to stress via their capacity to regenerate cardiac tissues by differentiating into cardiomyocytes and coronary vessels *(*Laugwitz et al., 2005, Nature, 433, 647-53*;* Limana et al., 2007, Circ. Res., 101(12), 1255-1265*;* Bearzi et al., 2009, Proc. Natl. Acad. Sci. USA., 106(37), 15885-15890*;* Angert et al., 2011, Circ. Res., 108(10), 1226-1237*).* These cardiac stem cells have been described as c-kit-positive, Islet1 (ISL1) progenitors, epicardial progenitors, side population progenitors, stem cell antigen 1 (Sca1) progenitors, progenitors generating cardiospheres, stromal stem cells from the adult bone marrow, mesenchymal stem cells, or endothelial progenitor cells. All these progenitor cells have demonstrated an ability to differentiate into cardiomyocytes under the appropriate culture conditions.

Stem cells cell replacement therapies making use of stem cell preparation include ESC derived from heart tissue as well as adult pluripotent cells such as endothelial progenitor cells, that are able to differentiate into contractile tissues, colonize the adult heart, thus promote repair and even reduce fibrosis. For example, endothelial progenitor cells, mononuclear bone marrow cells, mesenchymal stromal cells, cardiac muscle precursor cells, CD34-positive cells, c-kit-positive cardiac stem cells, stem cells isolated from the cardiac atrium, or cardiosphere-derived cells composed of c-kit-positive cells as well as cells expressing myocyte proteins, connexin 43, and CD105 can be used in cell-based tissue regeneration methods according to the invention for therapy of congestive heart failure and other chronic heart conditions.

Lung endogenous stem-progenitor populations have also been identified *(*Foronjy and Majka, 2012, Cells, 1(4), 874-885*;* Kubo, 2012, Stem Cells Transl. Med., 1(8), 627*).* These cells are important regulators of tissue repair and fibrosis. Maturation, aging, and senescence in diseased or damaged lung cells, including endogenous stem cells, affect the regenerative capacity of the organ. Therefore, for example, bone marrow derived mesenchymal stem cells or endothelial progenitor cells can be used in cell-based tissue regeneration methods to promote tissue repair and reduce fibrosis, and thus prevent acute injury, according to the invention for therapy of acute and chronic lung diseases, including chronic lung disease, pulmonary arterial hypertension, pulmonary fibrosis, chronic obstructive pulmonary disease (COPD), and emphysema.

Although the kidney is often regarded as an organ without regenerative abilities, a variety of resident renal cell populations that demonstrate stem cell characteristics have been identified in kidney and explain the recovery observed following acute injury *(*Acharya, 2012, J. Assoc. Physicians India, 60, 32-3*;* Yuen et al., 2012, Semin. Nephrol., 32(2), 215-23*;* McCampbell and Wingert, 2012, Biochem. J., 444(2), 153-68*).* Therefore, for example, those resident renal cell populations, in particular, renal tubular epithelial cells and several populations of extra-renal stem cells of bone marrow origin such as mesenchymal stem cells can be used in cell-based tissue regeneration methods according to the invention for therapy of acute or chronic kidney injuries causing renal lesions and fibrotic pathologies.

The liver is endowed with a great potential to reverse parenchymal tissue loss due to injury. Liver progenitor cells have been suggested to participate, along with hepatocytes proliferation, in liver tissue repair and homeostasis *(*Greenbaum and Wells, 2011, Int. J. Biochem. Cell. Biol., 43(2), 222-9*;* Cantz et al., 2008, Cell Tissue Res., 331(1), 271-82*;* Mancino et al., 2007, Ital. J. Anat. Embryol., 112(2), 93-109*).* The progenitor cell niche for liver repair comprises both resident liver stem cells and circulating stem cells from the bone marrow. Their participation is particularly in diseases affecting hepatocyte proliferation, such as chronic liver injuries. As liver fibrosis is an outcome of chronic liver injury of any etiology, and as liver repair mechanisms involving resident bone marrow-derived stem, notably bone marrow-derived fibrocytes and mesenchymal progenitors which are also collagen expressing cells, play an active role in liver fibrosis *(*Kisseleva and Brenner, 2012, J. Hepatol., 56(4), 965-72*),* methods and compositions according to invention are useful for the promotion of normal liver repair while preventing pathological fibrosis.

The terms "treat", "treating" and "treatment" and similar referents refer to obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes:
(a) preventing the disease from occurring in a subject who may be predisposed, but has not yet been diagnosed with the disease; for example based on familial history or on therapeutic history indicating a predisposition or risk to develop a disease or condition ("prophylactic treatment");
(b) causing a reduction of the severity of the condition or symptoms associated with the condition, or a delay or slowing down of the progression of the condition or symptoms associated with the condition, such as inhibiting the disease, i.e., arresting its development; or relieving the disease, i.e., causing regression of the disease and/or its symptoms or conditions such as improvement or remediation of damage ("therapeutic treatment"). In a particular aspect, the use of the terms treat," "treating" and "treatment" and similar referents refers to the obtaining a desired pharmacological and physiological effect in a subject through the administration of specific compounds in an effective amount or through the conduction of a specific method, while a subject is suffering from a condition.

The term "efficacy" of a treatment or method according to the invention can be measured based on changes in the course of disease or condition in response to a use or a method according to the invention. For example, the efficacy of a treatment or method according to the invention can be determined by measuring the recovery of organ function, for example, in the heart by echocardiography or angiography, or in the lungs by changes in airflow rates, lung compliance or gas exchange, or in many tissues by determining the level of fibrosis or presence of fibrotic lesions in a tissue for example by needle, forceps, or surgical tissue biopsies of myocardium, airways, lung, liver or kidneys, or by determining survival of transplanted cells, differentiation and function for example by immunochemical or genetic methods in tissue biopsies. According to another example, the efficacy of a treatment or method according to the invention can be measured by determining the level of cell maturation or of cell differentiation in the cell culture medium using standard methods in the art, including visual observation by microscopy, detection of markers which are specific for the targeted differentiated tissue by immunological staining or blotting and by molecular assays of mRNA, chromatin, nuclear DNA, mtDNA, or microRNA.

The terms "effective amount", "therapeutic effective amount", and "prophylactic effective amount" refer to a dosage of a compound or composition effective for eliciting a desired effect, commensurate with a reasonable benefit/risk ratio and will vary from subject to subject, depending, for example, on species, age, and general condition of a subject, severity of the side effects or disorder, identity of the particular compound(s), mode of administration, and the like. In certain embodiments, the desired dosage can be delivered using multiple administrations. This term as used herein may also refer to an amount effective at bringing about a desired *in vivo* effect in an animal, preferably, a human, such as induction of mitochondrial biosynthesis, induction of cell mobilization, and/or induction of cell maturation, and/or induction of cell differentiation, and/or induction of cell proliferation, and/or induction of cell survival, and/or prevention of fibrosis, and/or reduction of fibrosis.

The term "administration" or "administering" refers to delivery of a compound or composition by any appropriate route to achieve the desired effect. Administration may include any convenient route of administration, whether systemically/peripherally or at the site of desired action, including but not limited to, enteral (oral, e.g. by ingestion), intravenous, topical, pulmonary, parenteral, intramuscular, subcutaneous, rectal, vaginal, ophthalmic, and intraperitoneal routes.

The term Re-based CORMs and derivatives includes rhenium (Re)-based compounds as described in WO 2011/110315, which is incorporated herein by reference in its entirety. Re-based CORMs and derivatives may be designed and synthesized to contain a vitamin B12 or a derivative thereof as a ligand (L₁-L₄), allowing derivatives to be fully water soluble and biocompatible, and potentially acting as a mitochondrial targeting moiety. According to one aspect, Re-based CORMs and derivatives are designed and synthesized to contain conjugates for specific tissue targeting, notably heart-, lung-, or kidney-tissue targeting conjugates, According to one aspect, Re-based CORMs and derivatives are used with drugs or drug-like compound, organ, tissue or cell preservation agents or compositions, immunosuppressive, anti-inflammatory, antibiotic, antioxidant, anti-fungal compounds, blood thinning medications, insulin, as well as treatments to lower the blood pressure or heart rate, for example beta blockers, anti-arrhythmic medications, vasodilators, or agents to raise blood pressure or prevent shock, such as vasopressors. The CORMs and derivatives thereof according to the invention may be prepared according to methods described in WO 2011/110315 and Zobi et al., 2012, Dalton Trans., 41(2), 370-8*.*

According to one aspect, Re-based CORMs or derivatives thereof are capable of inducing expression of HO-1, in an amount effective to induce mitochondrial biosynthesis and thereby inducing or promoting mobilization, differentiation, maturation, proliferation, survival of stem cells with without damaging the existing mitochondria.

According to a particular aspect, Re-based CORMs and derivatives according to the invention have improved stability in aqueous aerobic media, are water-soluble, biocompatible, have very low toxicity, their byproducts are among the least toxic of all of the rare inorganic compounds.

According to another particular aspect, Re-based CORMs and derivatives according to the invention comprise ligands that target the Re-based CORM to specific tissues. In particular, Re-based CORMs and derivatives may contain a vitamin B12 ligand that targets the Re-based CORM to cells that utilize significant amounts of Vitamin B12. Further, as Vitamin B12 (Cyanocobalamin, Cbl) is used a co-factor primarily by two enzyme systems, one in the cytoplasm and one in the mitochondrion; thus Cbl can be potentially used as a cell targeting moiety *(*Gherasim et al., 2013, Biochimie, 95(5), 1023-32*).* While neither enzyme that requires Cbl is directly involved in the induction of mitochondrial biogenesis, Cbl-Re-based CORM conjugates are Re-based CORM derivatives according to the invention capable of release CO upon approach or entry into the mitochondria and thereby stimulate more efficiently mitochondrial biogenesis.

The invention relates to methods to treat or prevent a disease, promote tissue repair, and combat fibrosis, comprising administering Re-based CORMs or derivatives conjugated for specific tissue targeting. In particular, Re-based CORMs may be derivatized and/or conjugated to specific heart targeting molecules or ligands, such as nitrile and isonitrile ligands or other derivatives thereof. In a particular embodiment, Re-based CORM derivative according to the invention is a Re-based CORM derivative wherein two, three or four of the L₂-L₄ ligands from the Re-based CORMs according to the invention are isonitrile groups such as isocyanate groups. In a further embodiment, Re-based CORM derivative according to the invention is a Re-based CORM derivative selected from the following group: 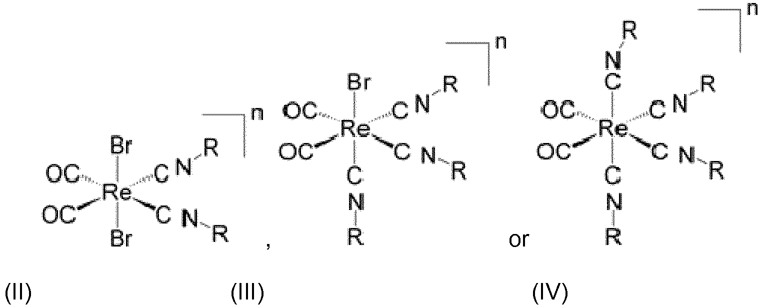 wherein R is an optionally substituted C1-C6 alkyl or an optionally substituted aryl and n is as defined herein..

An example of a Re-based CORMs of Formula (III)is the structure:

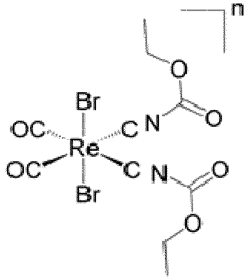

The term (mono-, bi-, tri-) "dentate" ligands refers to number of atoms in a single ligand that bind to a central atom in a coordination complex according to the invention. According to one aspect of the invention, L1 to L4 are monodendrate ligands.

The term "vitamin B12 or a derivative thereof" include cyanocobalamine molecule (henceforth vitamin B12 or simply B12) of the following formula: 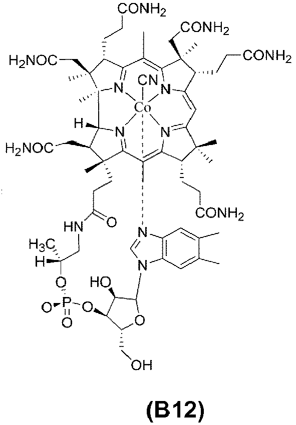 and any derivative thereof wherein the molecule of vitamin B12 or a derivative thereof can be conjugated to the Rhenium at different sites of conjugation which include, but are not restricted to, the cyano group (i.e. CN⁻), all amide groups around the corrin ring, the phosphate (i.e. PO₄⁻) or the hydroxide groups on ribose sugar moiety (e.g. 5' OH). For example, vitamin B12 derivatives include vitamin B12 with halogen-substituted corrin ring or lactone-type vitamin B12 derivatives such as described Wagner, 1965, Proc. Roy. Soc. A288, 344*,* optionally further substituted or lactone fragmented-type vitamin B12 derivatives such as described Fedosov et al. Biochemistry 50, 2011, 8090*,* optionally further substituted. Further examples of those derivatives have been described in Zobi et. Al, 2012, Dalton Trans., 41(2), 370-8*,* and Zobi et. al. 2013, J. Med. Chem., 56(17), 6719-31*.*

According to one aspect, a Vitamin B12 derivative is:

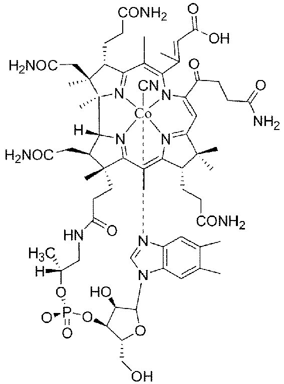

According to one aspect, the present invention relates to rhenium (Re)-based compounds of general formula (I): 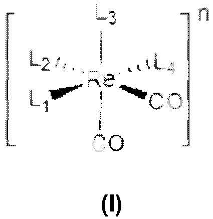 wherein Re is Re(I), Re(II) or Re(III), preferably Re(II); n is selected from 3- to 3+, such as 2-, 1-, 0, 1+ and 2+, in particular 1-, 0, 1+; L₁, L₂, L₃ and L₄ denote in each case independently of one another pharmaceutically acceptable monodentate, bidentate, tridentate ligands or combinations thereof.

In a specific embodiment, L₁, L₂, L₃ or L₄ independently selected from the group consisting of neutral or negatively charged monodentate ligands.. Non-limiting examples of suitable neutral ligands are imidazole, pyridine, including bi-pyridine, and pyrazine-type ligands, all being optionally substituted by one to four R as defined below. Non-limiting examples of suitable negatively charged ligands are cyanide, hydroxide and halogens, preferably hydroxide and halogen such as bromide and chloride.

In another specific embodiment, L₁, L₂, L₃ or L₄ are those ligands described in WO 2011/110315 (i.e. monodentate, bidentate, tridentate ligands or combinations thereof) and which contain a chemical functionality that allows attachment or conjugation to vitamin B12 or a derivative thereof at sites other than the CN- group. By "chemical functionality" are intended molecules independently selected from the group consisting of:
nitriles, isocyanides, primary, secondary and tertiary amines, such as primary and secondary amines, alcohols, carboxylic acids, phosphines, phosphates, phosphonites, sulfides, sulfoxides, alkyl, alkenyl, alkynyl, alkylidene, cycloalkyl, aryl, heteroaryl, arylalkyl, aryloxy, alkoxy, alkylthio, acyl, alkoxycarbonyl, acyloxy, acylamino, sulphonylamino, aminosulfonyl, alkylsulfonyl, diketone alkyl; each optionally substituted
by one to four R where possible;
wherein each R is independently selected from:
alkyl, alkenyl, alkynyl, alkylidene, cycloalkyl, aryl, arylalkyl, aryloxy, alkoxy, alkylthio, acyl, alkoxycarbonyl, acyloxy, acylamino, sulphonylamino, aminosulfonyl, alkylsulfonyl, carboxy, carboxamide, hydroxy, halogen, trifluoromethyl, nitro, nitrile and amino optionally mono-, di- or tri-substituted by alkyl, acyl or alkoxycarbonyl, wherein any of
where possible;
wherein each R"" is independently selected from:
alkyl, alkenyl, alkynyl, alkylidene, cycloalkyl, aryl, arylalkyl, aryloxy, alkoxy, alkylthio, acyl, alkoxycarbonyl, acyloxy, acylamino, sulphonylamino, aminosulfonyl, alkylsulfonyl, carboxy, carboxamide, hydroxy, halogen, trifluoromethyl, nitro, nitrile and amino optionally mono-, di- or tri-substituted by alkyl, acyl or alkoxycarbonyl, wherein any of where possible;
In a particular embodiment, each R is independently selected from
C₁₋₆ alkyl, C₂₋₆ alkenyl, C₂₋₆ alkynyl, C₂₋₆ alkylidene, C₃₋₇ cycloalkyl, aryl, arylalkyl, aryloxy, C₁₋₅ alkoxy, C₁₋₆ alkylthio, C₁₋₆ acyl, C₂₋₇ alkoxycarbonyl, C₁₋₆ acyloxy, C₁₋₆ acylamino, sulphonylamino, aminosulfonyl, C₁₋₆ alkylsulfonyl, carboxy, carboxamide, hydroxy, halogen, trifluoromethyl, nitro, nitrile and amino optionally mono-, di- or tri-substituted, preferably mono- or di-substituted by C₁₋₆ alkyl, C₁₋₆ acyl or C₂₋₇ alkoxycarbonyl.
In a specific embodiment, L₁, L₂, L₃ or L₄ is Cyanocobalamin.

In another specific embodiment, the rhenium (Re)-based compound according to the invention if of formula [Re^{II}Br₂(CO)₂L₂L₄].

In another further specific embodiment, the rhenium (Re)-based compound according to the invention if of formula [Re^{II}Br₂(CO)₂L₂L₄] and L₂ and L₄ are independently selected from water, methanol, chloride, bromide, imidazole, N-methylimidazole, pyridine, acetylacetonate, methoxyisobutylisonitrile, 2,2'-bipyridine, pyrazine and cyanide.

In a specific embodiment, L₄ is the following group:

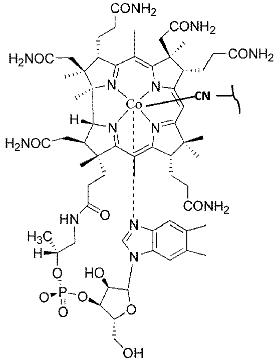

In another specific embodiment, L₄ is :

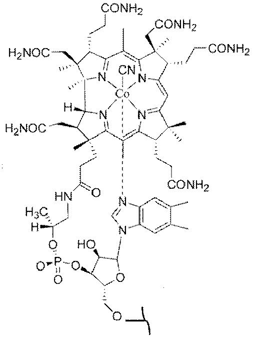

In another specific embodiment, L₄ is Cyanocobalamin and L₁, L₂, L₃, are as described herein and wherein Rhenium is conjugated to B12 so as to form a complex as described below: 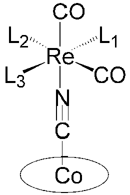 wherein the group 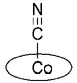 is

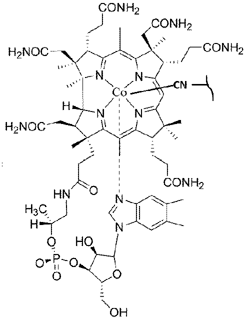

In another specific embodiment, the rhenium (Re)-based compound according to the invention is of the following formula:

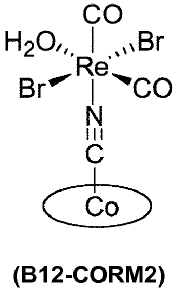

In one aspect, the invention provides a method of preparation of a cell composition for cell-based therapy comprising a step of inducing expression of heme oxygenase 1 (HO-1) in a stem, or a progenitor, or a precursor, or a totipotent, or a pluripotent, or a multipotent or an oligopotent, or a bipotent, or an unipotent cell.

In another aspect, the invention provides a method of preparation of a cell composition for cell-based therapy comprising a step of contacting cells, in particular stem cells, with a Re-based CORM, a derivative thereof or a mixture thereof.

In a further aspect, the invention provides a method according of the invention of preparation of a cell composition (for example useful for cell-based therapies aiming at replacing lost tissues or at repairing damaged tissues) comprising a step of contacting cells, in particular stem cells, with a Re-based CORM, a derivative thereof or a mixture thereof, in an amount effective to induce mitochondrial biosynthesis, display cytoprotective effects, and/or stimulate differentiation, maturation, proliferation, survival of cells and tissues to be transplanted while preventing fibrosis. According to one particular aspect, such method improves cell-based treatment, tissue regeneration and repair, and contributing in preventing fibrosis.

In another aspect, the invention provides a method for promoting tissue growth and/or repair, in particular for improving cell/tissue survival. In particular, the said method being an *in vitro* or an *ex-vivo* (in culture) method for promoting tissue growth and/or repair. According to another aspect, the said method being a method for promoting tissue growth and/or repair in a subject.

In a further aspect, the invention provides a method for promoting tissue growth and/or repair comprising a step of contacting said tissue with a Re-based CORM, a derivative thereof or a mixture thereof or administering a Re-based CORM, a derivative thereof or a mixture thereof, in an organ, tissue or a subject in need thereof of an amount effective to induce mitochondrial biosynthesis, stimulate regeneration, and/or combat fibrosis, in particular an amount effective to promote mobilization, differentiation, maturation, proliferation, survival of resident stem cells, while blocking or preventing fibrosis. According to one particular aspect, such methods improve cell/tissue survival and/or prevent fibrosis thereby improving tissue regeneration and repair.

In another aspect, the invention provides a method for treating or preventing fibrosis or fibrotic lesions in a subject, organ or tissue. In particular, such a method comprises administering a Re-based CORM, a derivative thereof or a mixture thereof in a subject, organ or tissue in need thereof of an amount effective to induce mitochondrial biosynthesis, stimulate regeneration, and/or combat fibrosis.

In a further aspect, those methods comprise contacting or administering a Re-based CORM or a derivative or a combination thereof, co-administered (prior to, simultaneously or sequentially) with stem cells or tissues or organs to mammals in need thereof.

Methods, uses and compositions according to the invention are useful in a broad range of applications including, for example, cell protection, cell differentiation, cell maturation, tissue growth (*in vitro, ex vivo, in vivo*), tissue protection, tissue repair, fibrotic disease or disorder prevention, and fibrotic disease or disorder treatment.

In certain aspects, methods, uses and compositions according to the invention provided are useful for promoting tissue growth and/or repair and for preventing development of fibrotic lesions following tissue grafting wherein Re-based CORM, a derivative thereof or a mixture thereof are directly contacted with or administered to cells or to an isolated tissue in culture before transplantation/grafting to a mammal in need thereof *(ex-vivo),* in an amount effective to display cytoprotective effects, and stimulate differentiation, maturation, proliferation, survival of cells and tissues.

In a particular aspect, cells used in a method or composition according to the invention are selected from non-human embryonic stem cells, human fetal stem cell, human neonatal stem cell, a human adult stem cell, human adult progenitor cell, human neonatal progenitor cell and induced pluripotent stem cells (iPSs).

In a particular aspect, fibrotic responses or diseases according to the invention are a constituent and a consequence of cardiac, pulmonary, renal, hepatic, and systemic disorders that lead to impairments in organ function.

In a particular aspect, diseases or disorders which may be treated or prevented by methods or compositions of the invention include those related to mitochondrial disorders, acute and chronic disorders provoking tissue lesions, and fibrotic disorders. The diseases and disorders may include, but are not limited to, acquired mitochondrial damage associated with tissue damage and/or fibrosis including, for example, cardiac, pulmonary, renal, hepatic, or systemic pathologies, as listed herein.

In a particular embodiment, diseases or disorders which may be treated or prevented by methods or compositions of the invention include dilated cardiomyopathies, congestive heart failure, anthracycline chemotherapy-drug induced heart failure, viral, toxic or idiopathic myocarditis, heart transplant rejection, and ischemic or complex forms of myocardial fibrosis such as obesity-linked heart failure or age-dependent cardiomyopathy, hypertensive cardiomyopathy, and cardiac hypertrophy.

In another particular embodiment, diseases or disorders which may be treated or prevented by methods or compositions of the invention include respiratory disorders and pulmonary lesions, such as acute lung injury (ALI), chronic obstructive pulmonary disease (COPD), chronic bronchitis, emphysema, cystic fibrosis, pneumonia, pulmonary edema, lung cancers, pneumoconiosis, interstitial lung disease (ILD), idiopathic pulmonary fibrosis, autoimmune lung diseases, lung transplant rejection, primary pulmonary arterial hypertension, secondary pulmonary arterial hypertension, pleural effusion, obesity hypoventilation syndrome, and neuromuscular lung disorders.

In another particular embodiment, diseases or disorders which may be treated or prevented by methods or compositions of the invention include acute and chronic kidney diseases, obstructive nephropathy, chronic kidney diseases, diabetes including diabetic renal injury, high blood pressure, immune system conditions affecting kidneys such as lupus or HIV/AIDS or hepatitis B or hepatitis C, pyelonephritis, polycystic kidney disease, kidney damage from certain drugs or toxins including overuse of NSAIDs (non-steroidal anti-inflammatory drugs), and atherosclerotic renal artery stenosis.

In another particular aspect, diseases or disorders which may be treated or prevented by methods or compositions of the invention include chronic liver diseases, cirrhosis liver lesions, passive or active fibrosis affecting for example the portal area or the intra lobule tissue or the center vein or the surroundings of the bile ducts or the Glisson capsule, fibrosis due to viral hepatitis, such as hepatitis B or C or D, to parasitic infections, to alcohol, to autoimmune disorders such as biliary hepatic fibrosis, to metabolic disorders such as Wilson's disease or liver lenticular degeneration or hemochromatosis, to alcoholic or other drug-induced liver fibrosis, as well as hepatic steatosis, primary biliary cirrhosis, primary sclerosing cholangitis, Budd-Chiari syndrome, hemochromatosis, alpha 1-antitrypsin deficiency, glycogen storage disease type II, transthyretin-related hereditary amyloidosis, Gilbert's syndrome, biliary atresia, alagille syndrome, or progressive familial intrahepatic cholestasis.

In another particular aspect, diseases or disorders which may be treated or prevented by methods or compositions of the invention include sepsis and sepsis-related conditions, which may lead to inflammatory foci, cell death, or other tissue lesions as well as a fibrotic response. Mitochondrial biogenesis was shown to be a key, early pro-survival factor in a model of bacterial-induced sepsis *(*Haden et al., 2007, Am. J. Respir. Crit. Care Med., 176(8), 768-77*).*

In another particular aspect, diseases or disorders which may be treated or prevented by methods or compositions of the invention include non-infectious systemic inflammatory response syndrome (SIRS).

The invention encompasses the administration of a Re-based CORM or a derivative thereof or a formulation thereof according to the invention wherein it is administered to a subject, organ or tissue prior to, simultaneously or sequentially with other therapeutic regimens or co-agents useful for tissue regeneration, for example for cardiac or skeletal muscle regeneration after immobilization or to hasten recovery from muscle weakness due to critical care myopathy or sarcopenia, and/or in preventing or treating fibrotic diseases or disorders or prior to, simultaneously or sequentially with surgical or medical intervention aiming at tissue regeneration and/or preventing or treating fibrotic diseases or disorders. According to the invention, the Re-based CORMs or derivatives thereof or formulations thereof according to the invention can be administered alone or in combination with a co-agent useful for inducing cell or tissue regeneration, in particular resident stem cell regeneration (e.g. multiple drug regimens), in a therapeutically effective amount.

In particular, the administration of a Re-based CORM or a derivative thereof or a formulation thereof according to the invention may be used before, after, or at the moment of organ transplantation, tissue or cell implantation, angioplasty, cardioversion, catheterization, balloon valvuloplasty, valve repair or replacement. Re-based CORMs or derivatives may also be used before, after, or at the moment of medical intervention such as bronchodilatation, ventilation, or dialysis.

In these combination treatments and interventions, the Re-based CORMs or derivatives may be considered as adjuvants, and the corresponding method of treatment as adjunct treatment. In combination with such compounds and/or interventions, the Re-based CORMs or derivatives treatment will provide additional benefits for a patient by increasing mitochondrial biogenesis, stimulating tissue repair and combating fibrosis.

According to another aspect, Re-based CORMs or derivatives thereof can be used alone or in combination with further compounds. Such compounds may be organ preservation agents or compositions, such as but not limited to, Euro-Collins solutions, Ross-Marshall citrate solutions, Bretschneider histidine tryptophan ketoglutarate solution, Phosphate-buffered sucrose solution, University of Wisconsin solution, Celsior solution, Kyoto ET solution, Custodiol, HTK solution, Koehler solution, SoStroSol solution, IGL-1 solution, SCOT solution, St Thomas solution, Plegisol™, cold Lactated Ringer (LR) solution, UW solution containing CO, curcumin-cyclodextrin complex (CDC) solution, extracellular phosphate-buffered solution type 4 (Ep4) solution, low-potassium dextran (LPD) solution. Such compounds may be drugs considered may be immunosuppressive drugs, including but not limited to, drugs of the group of cyclosporins (Neoral, Sandimmune, SangCya), of the group of azathioprines (Imuran), of the group of corticosteroids such as prednisolone (Deltasone, Orasone), as well as basiliximab (Simulect), daclizumab (Zenapax), Sirolimus (Rapamune), muromonab CD3 (Orthoclone OKT3), Mycopehnolate (CellCept), tacrolimus (Prograf), Glatiramer acetate (Copaxone). Drugs may also be anti-inflammatory medicines such as corticosteroids, antibiotics, antioxidants such as N-acetylcysteine (Mucomyst), anti-fungal treatment, treatments to lower the blood pressure in the pulmonary arteries such as prostanoids, including epoprostenol, treprostinil, or iloprost, or endothelin receptor antagonists, such as bosentan and ambrisentan, or phosphodiesterase inhibitors, like sildenafil, activated protein C, medications to relieve anemia, medications to lower cholesterol levels such as statins or fibrates, diuretics, calcium and vitamin D supplements, medications to lower the amount of phosphate in blood, high blood pressure medications such as angiotensin-converting enzyme (ACE) inhibitors or angiotensin II receptor blockers, beta blockers, blood thinning medications, such as daily aspirin therapy, anticoagulants, anti-arrhythmic medications, vasodilators, insulin, and vasopressors.

According to another aspect, the cells used in the methods according to the invention, notably stem cells mentioned above are used in a culture medium optionally comprising appropriate differentiation factors, the nature of which may be adapted to the nature of the cell. For example, neural stem cells can be used in a culture medium further comprising antagonists to TGF beta, dorsomorphin, as differentiation factors. In addition, heart or lung progenitor cells can be used in a culture medium containing fetal serum (FS), fetal serum protein extracts, myogenic regulatory factors (MRF), stem cell factor (SCF), insulin-like growth factor (IGF), leukemia-inhibitory factor (LIF), angiopoietin (Ang), colony stimulating factors (CSF), various interleukins (IL), and/or other pre-selected differentiation factors.

According to another aspect, the invention provides a cell preparation comprising Re-based CORMs, derivatives thereof or a mixture thereof combined with at least one co-agent useful in the preparation of stem cells, in particular differentiation factors, such as those listed, but not limited to [0089].

According to another aspect, Re-based CORMs or derivatives thereof may be combined with further components and/or pharmaceutically acceptable excipients to prepare standard pharmaceutical compositions. Pharmaceutically acceptable excipients include any and all solvents, diluents, or other liquid vehicles, dispersing and/or granulating agents, suspension aids, surface active agents, isotonic agents, thickening or emulsifying agents, preservatives, solid binders, disintegrating agents, buffering agents, lubricating agents, chelating agents, and the like, as suited to the particular dosage form desired.

Pharmaceutical compositions according to the invention include pharmaceutically acceptable oral formulations such as emulsions, microemulsions, solutions, suspensions, syrups and elixirs, solid and gelatin capsules, tablets, pills, powders, granules; topical formulations (including e.g. transdermal, intranasal, ocular, buccal, sublingual) as powders, ointments, creams, drops, ointments, pastes, creams, lotions, gels, powders, solutions, sprays, inhalants and/or patches; mucosal; pulmonary formulations (e.g. for intra-tracheal instillation, bronchial instillation, inhalation or insufflation therapy using, e.g. an aerosol, e.g. through mouth or nose) in the form of droplets of a solution and/or suspension or of coarse powder; rectal and vaginal (typically suppositories); parenteral formulations (e.g. for injection, including subcutaneous, intradermal, intramuscular, intravenous, intraarterial, intracardiac, intramedullary, intrathecal, intraspinal, intracapsular, subcapsular, intraorbital, intraperitoneal, intratracheal, intraventricular, subcuticular, intraarticular, subarachnoid, and intrasternal) as by acceptable emulsions, microemulsions, solutions, suspensions, syrups and elixirs, sterile injectable aqueous or oleaginous suspensions; ophthalmic formulations in the form of eye drops; depot formulation.

In certain embodiments, Re-based CORMs or derivatives thereof of the present invention are provided in an effective amount in the pharmaceutical composition. In certain embodiments, the effective amount is a therapeutically effective amount. In certain embodiments, the effective amount is a prophylactically effective amount.

As noted above, the methods and compositions disclosed herein can improve mitochondrial biosynthesis, maturation of cells such as, for example, stem cells and progenitor cells in a tissue, and thereby improve survival and prevent or reduce tissue fibrosis. Suitably, the methods improve mitochondrial biosynthesis and/or cell maturation and/or fibrosis while not causing or inducing damage to existing or intact mitochondria in the cell. In most embodiments at "therapeutic doses" of the compound of the invention, the agent does not cause or induce measurable damage to existing or intact mitochondria in the cell as for example evidenced by intact rates of cell respiration, ATP production, malate/pyruvate utilization, or MTT reduction. For instance, as shown in Example 9 and Figure 15 below, shows that Re-based CORM provides protection against cytomix-induced loss of cell metabolic signal in Hepa 1-6 liver cells as opposed to HO-1 substrate, hemin, or to another downstream product of HO-1, bilirubin, whose effects are related CO-releasing properties and not to mitochondrial or cytotoxicity. Direct and indirect methods for detecting mitochondria, determining the number of mitochondria, or the detecting the amount or degree of mitochondria biosynthesis are known in the art. One of skill in the art will be able to select any appropriate or suitable method for detecting mitochondria, such as those disclosed herein, in connection with the methods. Re-based CORMs and derivatives according to the invention, in particular B12-CORM2, specifically address tissue repair by interdependently activating stem cell differentiation, by inducing mitochondrial biogenesis, and by triggering anti-fibrotic effects, represent an innovative approach that may overcome the limitations treatments for heart, lung, liver, kidney failure and fibrosis have faced until now without causing or inducing damage to existing mitochondria in the cells or causing pan-suppression of the host immune response.

### EXAMPLES

The following abbreviations refer respectively to the definitions below:
**EB** (expanded blastocyte), **ES** (embryonic stem), **H9c2 cells** (rat cardiomyoblast derived cells, ATCC CRL1446), **E14** cells (C57BL6 mouse embryonic stem cells, American Type Culture Collection), **mD3** cells (mouse embryonic stem cell line D3), **hMEL1** (human embryonic stem cell line NIH registry #0139), **MLC2a** (myosin light chain isoform 2), **MFN-2** (mitofusin-2), **IMMT** (mitochondrial inner membrane protein), CS (citrate synthase), **Pdha1** (pyruvate dehydrogenase alpha 1), **cTnT** (cardiac specific isoforms of troponin T), cTnI (cardiac specific isoforms of troponin I), **TBST** (Tris-buffered saline and Tween 20), **ECL** (enhanced chemiluminescence), **MT-ND1** (mitochondrial-encoded NADH dehydrogenase subunit 1), **COII** (mitochondrial-encoded cytochrome oxidase subunit II), **OCT3/4** (octamer-binding transcription factor 3 / 4), **PBS** (phosphate buffered saline), **BSA** (bovine serum albumin), **MMLV** (Moloney murine leukemia virus), **CPCs** (cardiac precursor cells), **HO-1** (heme oxygenase 1), **NRF-1** (nuclear respiratory factor-1), **PGC-1α** (peroxisome proliferator-activated receptor gamma coactivator 1-alpha), **Tfam** (mitochondrial transcription factor A), **αSMA** (alpha smooth muscle actin), **Sox2** (transcription factor SOX-2), **ANP** (atrial natriuretic factor), **GATA4** (transcription factor GATA-4), **Mef2** (Myocyte-specific enhancer factor 2A ), **MLC2** (Myosin regulatory light chain 2), **Nkx2.5** (Homeobox protein Nkx-2.5), **TCA** (tricarboxylic acid), **COX** (cytochrome c oxidase), CFU (colony-forming unit).

### Example 1: Differentiation of embryonic cells lines toward functional cardiomyocytes

Re-based CORMs of the invention are tested for their ability of inducing myocardioblast stem cell differentiation and maturation into cardiomyocytes as described below.

*Chemicals and reagents.* All chemicals and reagents were purchased from Sigma (St. Louis, MO, USA), unless otherwise specified. The mitochondrial probes Mitotracker green, JC-1 reagent, MitoSox™ red, MTT, Alexa-488, and Alexa-595 secondary antibodies were purchased from Molecular Probes (Invitrogen, Carlsbad, CA). B12-CORM2 was synthesized as described in WO 2011/110315 and *Zobi et al., 2012,supra.*

*Culture and differentiation of H9c2 cells.* Rat cardiomyoblast-derived H9c2 cells (ATCC CRL1446) were grown in Dulbecco's modified Eagle's media (DMEM) containing 10% fetal bovine serum and 1% (v/v) antibiotic mixture at 37°C in a humidified atmosphere at 5% CO₂. Cells were induced to differentiate toward the cardiac-like phenotype as described by Menard et al., 1999, J. Biol. Chem., 274(41), 29063-70*.* Following decreases in growth factor concentration, myoblasts rapidly lose their characteristics and become multinucleated myotubes; sub-culturing was therefore performed before cell confluence to prevent spontaneous trans-differentiation into the skeletal phenotype. Differentiation was induced by culturing myoblasts in DMEM supplemented with 1% fetal calf serum. B12-CORM2 at 25 µM was added to the cells at days 1 and 3 with the media replaced every 2 days. The effects of the differentiating treatment were monitored at days 1, 3, 5 and 8. H9c2 cardiomyoblasts were transfected with the mCAT vector described in Suliman et al., 2007, J. Clin. Invest., 117(12), 3730-41*,* or sh-HO1 (Origene) using FuGENE-HD (Roche). Cells transfected with the pcDNA™3.3-TOPO® TA Mammalian Expression Vector (Invitrogen) lacking the insert were the controls. The transfected cells were subjected to differentiation as above.

*Culture and differentiation of murine E14 cells, murine D3 cells and human MEL1 cells.* These cell lines were independently grown at 37°C in 5% CO₂ on gelatin-coated substrates prepared by coating plastic tissue culture flasks with a 0.1% gelatin solution. Cells were maintained in a standard medium (Iscove's Modified Dulbecco's Medium, Invitrogen) plus 15% newborn calf serum, non-essential amino acids, and nucleosides stock. Leukemia inhibitory factor (LIF) (Chemicon) at a final concentration of 1,000 U/ml and 100 µM 2-mercaptoethanol were added to standard medium to prevent differentiation. For the induction of differentiation the hanging drop method such as described in Keller, 1995, Curr. Opin. Cell. Biol., 7(6), 862-9 was used. E14 cells were cultivated without LIF in hanging drops to form embryoid bodies (EBs) (600-400 cells per drop) for 2 days, then kept in suspension for 2 days, and finally plated on gelatin-coated, 96 well, single-culture dishes or single well slides. For the differentiation of ESC by B12-CORM2, cells were treated as for spontaneous differentiation, except that during the hanging-drop stage (days 1-2), 25 µM B12-CORM2 was added to the medium. B12-CORM2 was also added to the media on days 1, 2, 6, and 8. Rhythmically beating aggregates in EB outgrowths were observed daily by light microscopy to record the morphology and number of beating cardiomyocytes. Adhered EBs monitored daily under an inverted microscope for spontaneously beating foci from day 3 onward until day 13. In total, 3 × 96 EBs from E14 cells were monitored. To confirm that the increase in contracting EBs is HO-1/CO-mediated, E14 cells were transduced with Sh-HO1 vector or empty vector, differentiated as above and monitored by fluorescence microscopy.

*HO-1 silencing in E14 cells.* E14T cells were seeded at 40,000-80,000 per well in 6-well plates. Transfection of HO-1 pRS-shRNA (OriGene, TR504621) was performed using FuGENE 6 (Roche), followed by selection on 4 µg/ml puromycin. The negative control for the knockdowns was an ineffective scrambled 29-mer shRNA cassette in pRS Vector (shRnd) (OriGene). After incubation for another 24h, the medium was changed to standard media as above [00106]. To confirm HO-1 knockdown in the cells, real time RT-PCR was performed for HO-1 mRNA expression. The protocol for differentiation was carried out as above.

*RNA extraction.* Total RNA was extracted from undifferentiated, spontaneously differentiated and B12-CORM2-induced differentiated ESC (days 0-13) or from H9c2 cells. Total RNA was extracted using the RNAqueous-4 PCR kit (Ambion) according to the manufacturer's instructions. Samples were treated with DNase I (4 units, Ambion) for 2h at 37°C then inactivated with DNase inactivation reagent (Ambion). The RNA was reverse transcribed using the Reverse Transcription System (Promega).

*Real-time PCR.* Total RNA from undifferentiated and differentiated cells was isolated using UltraSpec RNA isolation reagent (Biotecx). The cDNA was prepared with a high-capacity cDNA archive kit (Applied Biosystems), according to the manufacturer's suggestions. Real-time RT-PCR primers for HO-1, NRF-1, Poly, PGC-1α, Tfam, COXII, Nkx2.5, GATA4, Mefc2, MLC2a, ANP, cTnI and 18S were purchased from Applied Biosystems and used according to the manufacturer's protocol. All reactions were conducted with the 7000 Sequence Detection System for 40 cycles. The results were analyzed using the 2-DeltaDeltaCt quantification method *(*Arocho et al., 2006, Diagn. Mol. Pathol., 15(1), 56-61*).*

*MtDNA copy number.* Total DNA was extracted from E14 ESC in the undifferentiated, spontaneously-differentiated, and B12-CORM2-induced states (days 0-13) using the Sigma DNA isolation kit. MtDNA copy number was quantified with RT-PCR on a 7700 Sequence Detector System (AB Applied Biosystems). Primers were designed for cytochrome b (cyt b) with ABI Probe Design software (Applied Biosystem). Amplifications were performed on 10 ng total mtDNA using PCR primers (cyt b-s and cyt b-as). One copy of linearized pGEMT-cyt b vector was used as a standard for simultaneous mtDNA quantification. The cytochrome b probe, 5' FAM-ttcctccacgaaacaggatcaaa-TAMRA 3', contained at the 5' end the FAM (6-carboxy-fluorescein) as a fluorescent reporter dye and the 3' end, the TAMRA (6-carboxy-tetramethyl-rhodamine) as a quencher dye selected from a highly conserved region of mouse cyt b gene. Serial dilutions of 105-110 copies standard cyt b plasmid were prepared to establish a standard curve. Samples were tested for mtDNA at 1:100 and 1:1000 dilutions. Samples were analyzed in triplicate and the number of mtDNA copies per ng total cellular DNA was determined by logarithmic expression compared with the standards.

*Western analysis.* Cell lysates were prepared from cell preparations (H9c2 or E14 cells) and equal amounts of protein (20-30 µg) separated by SDS-PAGE as described in Suliman et al., 2003, J. Biol. Chem., 278(42), 41510-8*.* Membranes were incubated with validated polyclonal rabbit Abs against MLC2a, MFN-2, IMMT, CS, Pdha1, desmin, Nebulin, cTnT, porin (Santa Cruz Biotechnology, Santa Cruz, CA), or anti-tubulin or β-actin (1:1000; Sigma). After five washes in TBST, membranes were incubated at 1:10,000 in HRP-conjugated goat anti-rabbit or anti-mouse IgG (Amersham). Blots were developed with ECL, proteins quantified on digitized images in the mid-dynamic range and expressed relative to tubulin, β-actin, or porin.

*Mitochondrial membrane potential.* Mitochondrial membrane potential was assessed by labeling cells with 5,5,6,6-tetrachloro-1,1,3,3 tetraethyl-benzimidazolylcarbocyanine iodide (JC-1; Molecular Probes, OR). Cells were incubated in 0.5 µmol/l JC-1 and 2 ng/mL Hoechst 33342 for 15 min, rinsed, and observed by confocal microscopy (Zeiss 410 LSCM, 620x). Cells were excited at 485 nm and emission detected in both the red and green channels. Red fluorescence indicates active mitochondria with high membrane potential through the formation of J-aggregates. Green fluorescence is representative of JC-1 remaining in its monomeric form in less active mitochondria.

*Mito Tracker and immunohistochemical staining, imaging, and mitochondrial ROS detection.* Live cells grown on coverslips were incubated in the media containing MitoTracker green (Molecular Probes, Invitrogen, Carlsbad, CA) at 250 nM for 30 minutes, followed by three washes in media and fixation with 4% formaldehyde in 1 × phosphate-buffered saline (PBS). The fixed cells were permeabilized with 0.2% Triton X100 and immunostaining performed on the following protocol. Mitochondrial ROS was measured using MitoSox™ Red (Invitrogen, USA), a live-cell permeant dye rapidly and selectively targeted to mitochondria. Once in the mitochondria, MitoSox™ reagent is oxidized by superoxide and exhibits red fluorescence (with excitation at 510 nm and emission at 580 nm). These cells were also stained with MitoTracker green.

A variety of embryonic or precursor cell types, including murine E14 and mD3 cells, human MEL1 cells, and rat H9c2 cells were grown in cell culture using standard techniques or in some experiments in single chamber slides (E14 and H9c2 cells). EBs were digested with 3 mg/ml collagenase II (Gibco, Grand Island, NY) for 15 minutes at 37°C. Undifferentiated, spontaneously-differentiated and B12-CORM2-treated mouse ESC (days 3, 7, 9 and 13) were titrated into a single cell suspension. Cells were re-suspended in E14 mESC differentiation media, plated onto gelatin-coated single chamber slides and incubated overnight at 37°C/5%CO2. E14 ESC were fixed in 2% formaldehyde (Sigma) for 1h, permeabilized for 30 min in 1% (v/v) Triton X-100 (Sigma), placed in blocking solution for 30 min and incubated with primary anti-MT-ND1 (1:100; Molecular Probes), anti-OCT3/4 (1:100; Santa Cruz Biotechnology), anti-sacomeric α-actinin (Abcam), anti-GATA4 (Santa Cruz) anti-Nxk2.5 (Cell Signaling Technology, Danvers, MA), anti MLC2a (Abcam) and antiCS (Molecular Probes) in PBS containing 1% BSA for 2 h at room temperature. After three washes, the cells were incubated with Alexa flour secondary antibodies followed by the nuclear probe 4',6-diamidino-2-phenylindole-2HCl (DAPI, Invitrogen).

Fluorescent images were obtained with a Laser Scanning Microscope 510 (Carl Zeiss Microimaging, Thornwood, NY) and analyzed using Zeiss Laser Scanning Microscope Image or imaged using Nikon Eclipse E600 fluorescent microscopy. ESC were visualized at 40x magnification with a dry lens and images captured on a Hamamatsu Digital Camera C4742-95. Data were collected using the IPlab 3.7 software (Nikon). Negative controls were used to determine appropriate exposure times and prevent false positive samples. For comparison, staining of ESC-derived cardiomyocytes was compared with established H9c2 cardiomyocytes using sacomeric α-actinin as a marker.

### Differentiation into cardiomyocytes

Successful differentiation was determined by morphological evidence, including an elongated shape, multiple nuclei, increased sarcomere staining for α-actinin, and a significant increase (more than twofold) in myosin light chain isoform 2 (MLC2a), a standard cardiac differentiation marker. To determine the role of HO-1/CO on myocardial differentiation, rat H9c2 cells, a myoblast line derived from embryonic heart tissue, were used to model differentiation during heart development by culturing in differential medium.

On the first day of culture in differentiation medium, no significant changes in morphology were found in either control or B12-CORM2-treated cells. On the third day of differentiation, cell elongation with the formation of myotubes was seen in B12-CORM2-treated cells, whereas myotubes were not observed in control cells. On the fifth day of differentiation, B12-CORM2-treated cells became multinucleated, but few multinucleated cells were observed in control cells. On the eighth day of differentiation, multi-nucleation was observed in both B12-CORM2-treated and control cells. These data indicated that the B12-CORMs treatment accelerated myotube formation and cellular differentiation.

Differentiation of mouse embryonic stem (ESC) E14 cell line is characterized by loss of pluripotency and concomitant activation of transcriptional programs for differentiation. A quantitative assessment of the effects of B12-CORM2 treatment on cell differentiation was performed in ESC using mRNA expression for the critical genetic pluripotency markers Oct4 and Sox2. As measured by real time RT-PCR, mRNA expression for these two progenitor genes was lost in a dose--dependent manner in E14 mouse ESC exposed to B12-CORM2 for 24h in the absence of leukemia inhibitory factor (LIF) (Table 1). B12-CORM2 treatment (25 µM, in the absence of LIF) also caused a time-dependent Oct4 and Sox2 mRNA down-regulation in E14 cells (Table 2). Further, the time-dependent response to B12-CORM2 was similarly demonstrated in other embryonic cell lines, notably mouse mD3 and human hMEL1 cells (Table 1). In addition, B12-CORM2 administration to E14 cells proved to increase respiration, as demonstrated with the MTT assay, which indicates the respiring mass of mitochondria as well as the viability of the cells (Table 3).

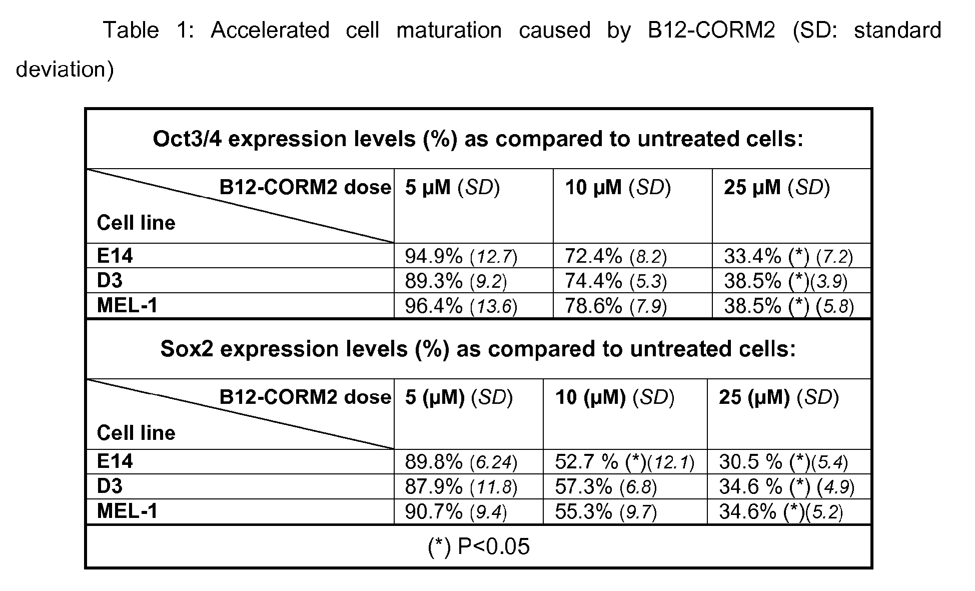

**Table 2: Time dependence of B12-CORM2 in E14 cells (SD: standard deviation)**

| **Expression levels (%) as compared to untreated cells:** | | | |
|---|---|---|---|
| | **B12-CORM2 time exposure** | | |
| | **12 h** (*SD*) | **1 day** (*SD*) | **3 days** (*SD*) |
| **Oct3/4** | 84.9% (*13.8*) | 33.4% (*) (*8.4*) | 4% (*) (*1.3*) |
| **Sox2** | 89.8% (*12.8*) | 30.5% (*) (*6.8*) | 3.8% (*) (*1.2*) |
| (*) P<0.05 | | | |

**Table 3: Increased MTT activity in E14 cells treated with B12-CORM2**

| **Increased MTT activity (%) as compared to untreated cells:** | | | | |
|---|---|---|---|---|
| **0 day** | **1 day** | **3 days** | **5 days** | **8 days** |
| 100 % | 102 % | 146 % (*) | 159 % (*) | 149 % (*) |
| (*) P<0.05 | | | | |

Differentiation into cardiomyocytes was accomplished by the standard practice of decreasing media serum content from 10% to 1%. One of the earliest features of differentiation in E14 cells is the induction of HO-1 and mitochondrial biogenesis, which is accelerated by treatment with B12-CORM2 (25µM; see Figure 1). Fig. 1A shows HO-1 mRNA induction at days 3-7 followed by induction of the nuclear-encoded mitochondrial TCA cycle enzyme, citrate synthase (CS), with or without B12-CORM2 treatment. Fig. 1 B illustrates the corresponding increase in mtDNA copy number in these cells. The requirement for HO-1/CO system is demonstrated in cells where HO-1 has been silenced using shHO-1. The active vector, but not the empty vector, blocked the responses. Moreover, the use of B12-CORM-2 with HO-1 silencing had a greatly diminished effect on all three signals, indicating that the operation of the Re-based CORM according to the invention is through the mitochondrial mechanism. To confirm this effect, the mRNA expression levels of NRF-1 (Fig. 1C), PGC-1α (Fig. 1 D), mtDNA polymerase (Pol y; Fig. 1E), mitochondrial transcription factor-A (Tfam Fig. 1 F) and mtDNA-encoded cytochrome oxidase subunit II (COII; Fig. 1 G) were measured with and without B12-CORM2 and with or without siHO-1. Thus, B12-CORM2 clearly induces mitochondrial biogenesis in an HO-1 dependent manner.

Furthermore, B12-CORM2 treatment of H9c2 cells induced an increase in immunofluorescence staining of cardiac myocytes for sarcomeric α-actinin of the assembled actin filament with clear sarcomeric cross-striation and cell size, after various differentiation protocols. Intercellular cardiomyocyte connections with mitochondrial traffic (mitotrail, visualized with MitoTracker green) indicate cell-cell metabolic cross-talk during cardiogenesis. All these changes did not occur when cells were transduced with sh-HO1 vectors: the α-actinin staining appeared as unassembled or disarrayed filaments or aggregates. Cells were also analyzed by confocal microscopy for mitochondrial mass (using Mitotracker green). The increase in mitochondrial mass, confirmed by the expression of the mitochondrial enzyme citrate synthase (CS), a mitochondrial matrix marker related to mitochondrial volume/mass, and up-regulation of TCA (Krebs) cycle components, e.g. pyruvate dehydrogenase (Pdha1), were especially prominent in cells treated with B12-CORM2. As shown below (see Figure 3), this effect was markedly decreased after transduction with mCAT or sh-HO1 vectors compared to control scrambled sh-RNA.

Differentiation of H9c2 cells in low serum medium is also associated with the formation of an expanded network of aligned mitochondrial networks and mitochondrial branching and increased expression of proteins involved in mitochondrial fusion and maturation of cristae, such as Mitofusin (MFN)-1 and -2 and the mitochondrial inner membrane protein (IMMT), suggesting that establishing an appropriate metabolic state is one of the earliest changes in fate that occurs when differentiation is initiated. Equal protein loading confirmation was determined by porin (VDAC) for mitochondrial proteins and tubulin for cytoplasmic and nuclear proteins. Consistent with the formation of an extensive mitochondrial network, differentiating H9c2 cells exhibited a gradual increase in mitochondrial function as determined by ND1 staining and ROS levels. Once again, MFN-2 and IMMT expression levels (examined by Western Blot analysis), as well as ND1 expression (examined by confocal microscopy), were up-regulated in B12-CORM2 treated cells, while loss of HO-1 expression in H9c2 cells resulted in a discontinuous mitochondrial network. Thus, transfection of mitochondrial-targeted catalase did not improve ATP production but significantly decreased mitochondrial H₂O₂ release (measured with MitoSox™ Red).

B12-CORM2 induces early E14 stem cell differentiation into beating cardiomyocytes E14 ESC not only lose pluripotency during differentiation (as shown by the loss of OCT4 and Sox2), but there is a concomitant activation of transcriptional programs for differentiation that results in the expression of cardiac-specific transcription factor genes and cardiac structural genes and proteins preceding functional activation (beating cells). In order to assess the impact of B12-CORM2 on the activation of redox-sensitive, cardiac-specific transcription factor genes and cardiac structural proteins in connection with the induction of HO-1 and mitochondrial biogenesis, cultures were supplemented with 25 µM of B12-CORM2 on days 2, 6, and 8 and EB out-growths were counted by microscopy whether or not they contained beating foci, on day 3, 7, 9, 11, and 13 after plating. B12-CORM2 induced the transcriptional program for mitochondrial biogenesis as shown above by an up-regulation of mtDNA replication at the onset of differentiation (Fig. 1 B), and mtDNA copy number increased dramatically to 273 copies per cell by day 7 (P<0.001). Expression of HO-1 corresponded to the expression of mRNA for GATA4, Nkx2.5, and Mef2c transcription factors as well as the canonical cardiac markers MLC2a, troponin I (cTnI) and atrial natriuretic peptide (ANP) commencing by day 3. The differentiation status with and without B12-CORM2 was established by examining the mRNA levels for these cardiac-specific genes by real time RT-PCR and cells that expressed at least two cardiac-specific antigens were considered positive for differentiation. In the absence of B12-CORMs, MLC2a, cTnI and ANP mRNA were expressed during differentiation of ESC, but with different timing (Fig. 2A-C). These expression levels were amplified in B12-CORM2-treated ESC. The up-regulation of MLC2a, cTnI and ANP mRNA was preceded by the progressive and significant increase in the expression of the cardiogenic transcription factors Nkx2.5, Mef2c, and Gata4 (Fig. 2D-F), indicating that significantly earlier cardiomyogenesis was induced by B12-CORM2. Real-time RT-PCR monitoring of expression levels at days 3, 7, 11 and 13 of differentiation confirmed that the expression of these cardiac-specific genes was significantly decreased in cultures having decreased HO-1 expression or activity. B12-CORM2 partly rectified the marked decrease in Nkx2.5, cTnI, and MLC2a expression in cultures transduced with sh-HO1 vector. The immunofluorescence analysis of the subcellular distribution of Nkx2.5 and GATA4 revealed that Nkx2.5 and GATA4 initially appeared as diffuse cytoplasmic staining in these cells at day 2, without nuclear localization. However, at day 13 of co-culture with B12-CORM2, most of the cells contained strong Nkx2.5 and GATA4 staining in the nucleus, and the number of cells with nuclear Nkx2.5 and GATA4 staining increased along with strong positive staining for the cardiac marker MLC2a. In cultures deficient in HO-1, little Nkx2.5 expression was evident and Gata4 displayed an altered cellular location and presented a mostly cytoplasmic staining pattern. Therefore, B12-CORM2 enhanced the nuclear translocation of redox-sensitive cardiac transcription factors and execution of the cardiogenesis program.

The differentiation of mouse ESC in vitro to a cardiomyocyte phenotype was assessed by tracking the appearance of well-synchronized rhythmically beating EB out-growths. Using an inverted microscope to monitor for spontaneously beating foci, it was shown that B12-CORM2 improved the differentiation of these cells. Fig. 3A shows that B12-CORM2 treatment increased the percentage of beating EB out-growths at day 7 from 15% in untreated control cells to 45% in B12-CORM2 treated cells. The percentage of beating EBs on day 13 reached 80% in treated cells compared with 50% in control cells (Fig. 3A, B). To confirm that this increase in contracting EBs required HO-1 induction, ESC were transduced with sh-HO-1 vector, which resulted in a far smaller number of beating foci compared with control cells (Fig. 3A). Also, the use of cells that are unable to express Tfam prevents the appearance of the mature EB phenotype (Fig. 3B). Further, the effect of B12-CORM2 on cardiomyocyte maturation depends on the presence of the intact mitochondrial DNA transcription factor, Tfam. In other words, B12-CORM2 only slightly rescued the decrease in percentage of beating foci caused by transfection with sh-HO-1 and failed to rescue the Tfam deficient cells.

In order to establish the phenotypic characteristics of the EB out-growths, the expression of cardiac-specific proteins was assessed at day 13. B12-CORM2-treated beating cells expressed larger amounts myocardial proteins, notably of troponin I (cTnl), myosin light chain 2V (MLC-2v), and myosin heavy chain (α-MHC) proteins on day 13 compared with untreated control cells, as measured by Western blotting with antibodies against those cardiac-specific proteins. The commitment to a cardiomyocyte fate was also assessed by evaluating the beating colonies for nebulin, a skeletal muscle protein that was not detectable in the beating EBs. In addition, cells cultured in B12-CORM2 also displayed myosin filaments characterized by the banded continuous-filament pattern, i.e. the classic striated appearance of cardiomyocytes, as imaged by staining for sarcomeric α-actinin, a special marker for mature cardiomyocytes showing adult myofibril organization. As expected, B12-CORM2-induced increases in cTnI were prevented by Sh-HO1 transfection. Loss of HO-1 expression also disrupted mitochondrial biogenesis in these cells and resulted in a low number of beating EBs. Similarly, the effect of B12-CORM2 on cardiac differentiation was blocked by the insertion of mitochondrial-targeted catalase (mCAT) and treatment with the mitochondrial antioxidant, MitoQ (Fig. 3C). These results indicated that the B12-CORM2 effect, which was observed at day 13 on the percentage of beating EB, depended on the earlier production of mitochondrial reactive oxygen species (ROS). Indeed, when MitoQ was given in the initial five days of differentiation, the B12-CORM2 effect was blocked (Fig. 3D). Finally, the use of JC-1, a specific marker of mitochondrial membrane potential to identify mitochondrial proliferation by confocal microscopy, clearly indicates a differentiated cytoplasm marked by increasing mitochondrial content as the cells progress through the process of differentiation. The presence of both high membrane potential (red fluorescence through the formation of J-aggregates) indicating active mitochondria and low membrane potential (green fluorescence representative of JC-1 remaining in its monomeric form) indicating less active mitochondria, in the merged images (orange/yellow fluorescence) indicates that these cells are both viable and respiring, but seem to be expressing mitochondria in different metabolic states. EB treatment with B12-CORM2 further promoted differentiation and significantly increased the high mitochondrial membrane potential population.

Taken together these data indicate that B12-CORM2 induces embryonic and precursor cell differentiation and accelerates mitochondrial maturation in myocardioblasts, in a HO-1-dependent manner. They show that B12-CORM2 is able to induce early E14 stem cell differentiation into cardiomyocytes through HO-1 induction of the transcriptional program for mitochondrial biogenesis and to amplify the early induction of myocardial differentiation markers. Further, the B12-CORM2-differentiated cells exhibit the main characteristics of cardiomyocytes, with an intact contractile apparatus.

### Example 2: Differentiation of primary cardiac precursor cell cultures

Re-based CORMs of the invention are tested for their ability to induce differentiation of primary cardiac precursor cells in culture as described below.

*Mice.* All animals were maintained in accordance with the recommendations of the US National Institutes of Health Guide for the Care and Use of Laboratory Animals (National Institutes of Health publication 86-23, 1985) and the experiments were approved by the Swiss animal welfare authorities. Neonatal C57BL/6 pups (1-2 days) were purchased from Janvier (Le Genest-Saint-Isle, France) and were housed in the animal facility until sacrifice.

*Cell isolation and culture.* Ventricles isolated from neonatal hearts (1-2 days old) are digested in buffer containing 0.5 mg/ml collagenase (Worthington Biochemical) and 0.45 mg/ml pancreatin (Sigma). Cardiomyocytes and cardiac non-myocyte cells (NMCs) are then separated by two differential platings (45 min). Adult ventricles isolated from adult hearts are digested in buffer containing 1 mg/ml collagenase (Worthington Biochemical) and 1 mg/ml pancreatin (Invitrogen Life Technologies). After enzymatic digestion, cardiomyocytes and non-myocyte cells (NMCs) are separated based on the capacity of NMCs to adhere to plastic. Neonatal or adult adherent cells, reflecting the NMC fraction, are expanded in culture in a proliferation medium consisted in a 3:1 mixture of DMEM and Medium 199 (Invitrogen life Technologies) supplemented with 10% horse serum (Oxoid), 5% fetal bovine serum (FBS) (Invitrogen Corp), 100 U/ml penicillin G, and 100 µg/ml streptomycin (Invitrogen Corp.), and supplemented with 1 µM dexamethasone (Sigma-Aldrich), 50 µg/ml ascorbic acid (Sigma-Aldrich), and 10 mM β-glycerophosphate (Sigma-Aldrich). Control medium consists of MEM plus FBS plus penicillin and streptomycin. All the media are changed twice a week.

*Treatments with rhenium-based compound of the invention B12-CORM2.* The effect of 10, 25, 50 or 100 µg/ml B12-CORM2 on neonatal NMCs is tested 2, 7, 14 and 21 days after isolation in either a differentiation *(*Rosenblatt-Velinet al., 2005, J. Clin. Invest., 115, 1724-33) or the control medium. B12-CORM2-treated cells differentiation status into cardiomyocytes is compared with untreated NMCs.

*Quantitative real time PCR.* Total RNA is isolated from the cells using Trizol reagent (Invitrogen Corp.). First-strand synthesis of cDNA from purified RNAs (2 µg/20 µl) are performed using oligo(dT)15 primers (10 µM, Microsynth) and the MMLV-reverse transcriptase (Promega Corporate, Wisconsin, USA). Resulting cDNAs are subjected to quantitative real-time polymerase chain reaction, performed in duplicates, using the SYBR Premix Ex Taq polymerase (Takara Bio Inc.) on a ViiA™ 7 Instrument (Applied Biosystems) Cycling conditions consist of an initial denaturizing step at 95 °C (10 min) followed by 40 cycles of a thermal step protocol consisting of 95 °C (1 s), 60 °C (20 s). Quantitative PCRs between control and treated cells are performed to determine mRNA expression levels for genes coding for BNP (Mm00435304-g1, Applied Biosystems), Gata-4 (MM00484689-m1, Applied Biosystems), Mlc-2v, Nanog, Nkx2.5 (Mm00657783-m1, Applied Biosystems), NPR-A, NPR-B, Sca-1, Troponin T (Mm00437164-m1, Applied Biosystems), α and β MHC (Mm00440354-m1 and Mm00600555- m1, Applied Biosystems), 18 S (sequences of primers reported in the Table 4 below, as described in Applied Biosystems references above and in Rosenblatt-Velinet al., 2005, J. Clin. Invest., 115, 1724-33), as well as eNOS, von Willbrand Factor, α smooth muscle actin, smooth muscle myosin heavy chain, and ANF.

**Table 4**

| **Gene** | **sense** | **antisense** | **Product size (bp)** |
|---|---|---|---|
| BNP | CTCCTATCCTCTGGGAAGTC | CTTGAGACCGAAGGACTCTT | 161 |
| Gata-4 | CTGTCATCTCACTATGGGCA | CCAAGTCCGAGCAGGAATTT | 259 |
| Mlc-2v | CACCCACCCATGCTAGTCTT | AATTGGACCTGGAGCCTCTT | 163 |
| Nanog | CACCCACCCATGCTAGTCTT | ACCCTCAAACTCCTGGTCCT | 152 |
| Nkx2.5 | CAAGTGCTCTCCTGCTTTCC | GTCCAGCTCCACTGCCTTCT | 130 |
| NPR-A | TCATGACAGCCCATGGGAAA | CGGTACAAGCTCCCACAAAT | 198 |
| NPR-B | CAAGTGCTCTCCTGCTTTCC | GGTGACAATGCAGATGTTGG | 209 |
| Sca-1 | ATGAGACGGTGGTGGGTTT | ACCCAGGATCTCCATACTTTC | 159 |
| Troponin T | GCGGAAGAGTGGGAAGAGACA | CCACAGCTCCTTGGCCTTCT | 127 |
| β MHC | ATGAGACGGTGGTGGGTTT | CTTTCTTTGCCTTGCCTTT | 117 |
| α MHC | ACTTTTGGGGCCTTCGTGTC | ACTCCGTGCGGATGTCAA | 130 |
| 18 S | ACTTTTGGGGCCTTCGTGTC | GCCCAGAGACTCATTTCTTCTTG | 96 |

*Nkx2.5 immunostaining.* In order to identify cardiac precursor cells (CPCs), Nkx2.5 immunostaining is performed on NMCs. Cells are incubated with a rabbit anti-Nkx2.5 (1/50 to 1/150) (Santa Cruz Biotechnology Inc) in the presence of biotin. Biotin anti-rabbit (1/150) (Jackson Immuno Research) and Cy3-Streptavidin (1/900) (Jackson Immuno Research) is applied to reveal Nkx2.5⁺ cells. DNA is stained with DAPI (Invitrogen Corp.). Nkx2.5 staining is also co-localized with other antigens, for example Troponin I or laminin to identify newly formed mature cardiomyocytes and to evaluate cardiomyocyte structure.

*Detection of cell apoptosis and necrosis.* Apoptosis and cell necrosis are estimated at multiple time points by flow cytometry analysis thanks to Annexin V (Enzo Life Sciences, 1/40) and Propidium Iodide (Enzo life Sciences, 1 µg/ml) stainings.

*Detection of Hypertrophy.* Quantitative PCR based on β and α MHC, ANF gene expression is used to determine the status of the hypertrophy program. Hypertrophy is demonstrated in mouse heart using LV + septal mass and cardiac to body weight ratios.

B12-CORM2 stimulates the differentiation of primary cardiac precursor cell cultures In order to assess the potential for B12-CORM2 to induce and/or increase CPC differentiation into cardiomyocytes, undifferentiated precursors from the cardiac NMC population of neonatal hearts are isolated, expanded in culture, and induced to differentiate into functional cardiomyocytes or endothelial cells or myofibroblasts. In parallel, the effects of B12-CORM2 on beating frequency, on cell survival, and on the development of hypertrophy, are assessed on mature neonatal beating cardiomyocytes as described above.

### Example 3: Differentiation of adult human cardiac progenitor cell lines

B12-CORM2's impact on the differentiation of human precursor cells into cardiomyocytes was assessed on well-characterized endothelial cardiac progenitor cells, isolated from human peripheral blood, as previously described *(Rignault-Clerc, et al., 2002, Burns).* Analyses carried out are described in Example 2.

### Example 4: Restored differentiation, mitochondrial integrity, and survival of embryonic rat cardiomyocytes affected by doxorubicin treatment

*Cell culture and treatments.* H9c2 cells (embryonic rat cardiomyocytes; ATCC) were maintained in DMEM (Invitrogen) supplemented with 10% fetal bovine serum (Invitrogen) and 2 mmol/I I-glutamine. Cells were plated on 35- or 100-mm dishes in DMEM with 2% FCS for differentiation. Doxorubicin (DOX) was added to complete medium and incubated at 37°C for different intervals at a concentration (0.5 µg/ml) similar to the plasma concentrations encountered in clinical use. Some cells were exposed to B12-CORM2 (25µM) with or without DOX. Cells collected after 3 days of differentiation.

*Differentiation assessment.* Western blot analysis and densitometry were performed relative to tubulin for alpha-actinin as a specific marker of cardiomyocyte differentiation and intactness of the contractile apparatus.

*Mitochondrial integrity and density assessment.* Western blot analysis and densitometry were performed relative to tubulin for citrate synthase.

*Cell survival.* Cell survival was measured by Trypan blue exclusion.

### B12-CORM2 rescues H9c2 cells from DOX-induced toxicity

B12-CORM2 treatment improved differentiation and survival of H9c2 cardiomyocytes exposed to DOX. DOX and/or B12-CORM2 were added complete medium on day 1. After 3 days of differentiation Western blot analysis and densitometry relative to tubulin revealed restored alpha-actinin expression, a specific marker of cardiomyocyte differentiation, and intactness of the contractile apparatus (Table 5). Furthermore, Western blot analysis and densitometry for citrate synthase (CS) enzyme, a marker of protection of mitochondrial integrity and density, showed that B12-CORM2 protected mitochondrial biogenesis (Table 5). Mitochondrial protection was accompanied by improved cell survival of B12-CORM2-treated cells exposed to DOX, as assessed by Trypan blue exclusion (Table 5).

**Table 5**

| **mRNA expression levels (% as compared to untreated cells):** | | | |
|---|---|---|---|
| | **+ B12-CORM2 (*SD*)** | **+ DOX (*SD*)** | **+ B12-CORM2 + DOX** (*SD*) |
| **Alpha actinin** | 172.2 % (*) (*27.1*) | 57.1 % (*11*) | 136 % (*) (*22.2*) |
| **Citrate synthase** | 133.1 % (*25.3*) | 60 % (*12.3*) | 114.2 % (*) (*13.1*) |
| **Surviving cells** | 115 % (*21.2*) | 61 % (*9.9*) | 88 % (*12.6*) |
| (*) P<0.05 | | | |

### Example 5: In situ differentiation of resident cardiac precursor cells in vivo and increased cardiac repair in mice with DOX-induced cardiac lesions

*Animal models.* WT C57BL/6 mice were purchased from Charles River Laboratories and housed under standard conditions. Doxorubicin (DOX) cardiomyopathy was induced by injecting DOX 15 mg/kg subcutaneously once per mouse. B12-CORM2 was administered subcutaneously once daily (1 to 2 mg) for three days. Mice were then followed for 7 or 14 days.

*Physiological and echocardiographic parameters.* In order to understand the physiological effects of the compounds of the invention on the mouse heart, body weight and heart weight, blood pressure, heart rate, cardiac dimensions and echocardiographic functional parameters (transthoracic echocardiography), ventricular wall and septum thicknesses, chamber dimension, and fractional shortening were measured.

*In vivo cardiac cell proliferation.* BrdU was administered (1 mg/ml for 96h in drinking water containing 5% glucose) before sacrifice. Expression of appropriate cardiac marker genes is documented using cardiac aurora kinase, GATA4, Nkx2.5, α and β MHC, MLC2a, MLC2v, Mef2c, Troponin I, α-actinin, c-kit, Islet-1, and Sca1 expression analysis, by semi-quantitative and quantitative RT-PCR analysis as well as by immunohistological analysis.

*Cardiac* fibrosis. Development of cardiac fibrosis was assessed using tissue staining for collagen van Gieson staining or with Masson's trichrome alone or in combination with the Verhoeff elastin stain (black) in order to differentiate small arteries and veins.

*Mitochondrial biogenesis.* The induction of mitochondrial biogenesis is assessed by examining changes in the nuclear accumulation and nuclear tissue staining of NRF-1 and PGC-1α by nuclear Western blot analysis and/or by immunohistochemical staining. The distribution of mitochondrial biogenesis is examined in cells and tissues for changes in citrate synthase (CS) enzyme content by Western blot analysis and/or by changes in mtDNA content (copy number) assessed by PCR.

*Apoptosis.* Apoptotic cell death in the heart tissue is studied by at least two of the following methods: Western blotting for active caspase 3 cleavage; *in situ,* by TUNEL assay (R&D Systems), myomesin staining or localization of single-stranded nuclear DNA staining.

*Mitochondrial oxidative phosphorylation and reactive oxygen species generation.* The adequacy of mitochondrial oxidative phosphorylation is assessed in two ways. In mitochondria isolated from cells and tissues by Ficoll gradient centrifugation, the organelles are studied in respiration media in a closed thermostatic cuvette at 35-36°C containing a miniature PO2 electrode. The rate of loss of PO2 from the cuvette is directly proportional to the rate of respiration (O₂ consumption). Respiration is measured per 0.5 mg mitochondrial protein in the presence of Complex I substrates (5 mM each of glutamate and malate) or Complex II substrate (10 mM succinate) with or without ADP (0.5 mM). The resting respiration (State 4) is the O₂ disappearance rate without ADP and the maximum respiration rate (State 3) is the O₂ disappearance rate with ADP. The respiratory control ratio (RCR) is State 3/State 4.

In intact cells, 500,000 to 1,000,000 cells in standard media are permeabilized in 0.05% digitonin and then placed in the thermostatic cuvettes. The substrates and ADP are then added and the States 3, 4, and RCR are measured as above. Also in cells, cells were incubated in a 96 well plate and add 10 microliters from a 12 mM stock of MTT and measure the change in Optical Density at 570 nm as the formazan is generated. The change in MTT reduction over time is related to the total mitochondrial respiration rate.

### In vivo impact of B12-CORM2 on cardiac differentiation

When B12-CORM2 is administered intravenously or by the other routes as mentioned above (1 to 10 mg), cardiac precursor proliferation is clearly detected by staining for the early cardiac marker Nkx2.5, by c-kit (stem cell factor receptor) staining for hematopoietic stem cells, as well as by Sca-1 (stem cell antigen-1) staining. Furthermore, an increase in co-staining for Sca-1 and markers for mitochondrial biogenesis, such as NRF-1 and PGC-1α, following B12-CORM2 treatment, is observed when compared to control mice.

*In vivo* impact of heart-targeting B12-CORM2 derivatives on cardiac differentiation The effects of B12-CORM2 and of heart-targeting B12-CORM2 derivatives on cardiac differentiation and repair potential are assessed and compared in wild-type newborn and adult mice. The efficiency of heart-targeting B12-CORM2 derivatives to stimulate cardiac differentiation following systemic administration is demonstrated, without evident carcinogenic effects.

### In vivo impact of B12-CORM2 or heart-targeting B12-CORM2 derivatives in mice with DOX-induced cardiac lesions and mice with cardiac lesion caused by a transverse aortic constriction, as compared to wild-type mice

Similarly to wild-type mice, B12-CORM2 treatment leading to CPC differentiation into cardiomyocytes, allows the repair and maintenance of cardiac integrity, and has a positive impact on DOX-induced cardiac lesions *in vivo.* B12-CORM2 and heart-targeting derivatives were assessed for their ability to limit the development of cardiac dysfunction, cardiac hypertrophy and dilation (echocardiography measures), susceptibility to pressure overload, apoptosis, as well as decline in mitochondrial oxidative phosphorylation and reactive oxygen species generation in these models. Three days of B12-CORM2 therapy efficiently led to cardiac repair in DOX-injured mouse heart, as demonstrated by a significant increase in the expression in cardiac cell proliferation markers aurora kinase and Nkx2.5 despite DOX administration (Fig. 4).

### Example 6: Sepsis model

*Animal models. S. aureus* (clinical isolate Rosenbach Seattle 1945; ATCC [Manassas, VA] #25923; 1 x 10⁶ to 10⁸ CFU) was prepared, counted, and implanted abdominally in fibrin clots into WT mice under anesthesia. Genetically-altered mice with conditional knockout of heme oxygenase 1 (HO-1) in the heart, lungs, or liver were studied to determine the need for this enzyme to be activated by the B12-CORM2 for its mode of action. Mice were killed at the indicated times and hearts, livers, lungs were harvested immediately for RNA, mtDNA, or for the preparation of mitochondrial and/or nuclear protein. Mitochondrial and nuclear pellets were stored in RIPA buffer at -80°C. Mice were also fluid resuscitated with 1.0 ml of 0.9% NaCl solution and treated with Vancomycin (3 mg/kg) in order to better simulate clinical conditions.

*B12-CORM2 administration.* In the sepsis mouse models, 3 consecutive injections of 30 mg/kg of B12-CORM2 (every 24h) were performed subcutaneously in groups of at least 4 mice per time point. Controls received vehicle alone (5% DMSO in 0.9% NaCl) or vehicle plus an inactive control (B12-ReAA) which is a Re-based complex of chemical formula C₆₆H₉₂BrCoN₁₄O₁₈Pre or NaReO₄ which is the final aerobic metabolite of B12-CORM2.

*Analyses.* Endpoint analyses for the activation of mitochondrial biogenesis, for the inflammatory response, and for evidence of tissue damage were obtained at 6, 24, 48 and/or 72 hours after sepsis depending on the endpoint chosen. In the survival studies, the number of living mice was assessed every 12 hours for 7 days. Statistical analysis was by ANOVA.

B12-CORM2 protective potential in a model of systemic infection

The mouse model of sepsis in which a fibrin-clot impregnated with live bacteria recapitulates many of the features of human peritonitis. This model presents a bacterial dose-dependent organ damage and mortality (Fig 5) and recapitulated many of the features of human peritonitis. B12-CORM2 appeared to provide a survival advantage (Fig 5), while inactive control drugs did not lead to any improvement in survival. The compound of the invention induced more robust survival responses including HO-1 and pAkt/Akt in the lungs and other tissues than did the infection alone. No B12-CORM2-mediated toxicity was observed as revealed by assessing LDH release by dying cells, trypan blue exclusion, and MTT assays.

### Example 7: B12-CORM2 stimulates mitochondrial biogenesis in vivo

Re-based CORMs of the invention have been documented for their ability to stimulate mitochondrial biogenesis *in vivo* as follows:

*Mice.* Young adult male C57BL/6J mice were purchased from Jackson Laboratory (Bar Harbor, ME). Genetically engineered mitochondrial green fluorescent protein (Mt-GFP) mice were obtained from Tokyo Metropolitan Institute of Medical Science, Tokyo, Japan, and bred at the Duke University animal facility. The studies were conducted under protocols approved by the Duke University (Durham, NC) Institutional Animal Care and Use Committee.

*B12-CORM2 administration.* The Mt-GFP mice were injected subcutaneously, intraperitoneally (25 or 50 µM/kg), or intravenously (at any of a range of doses between 1 and 11 µM/kg or up to 500µg or 3 x 50µg/day), in order to detect changes in tissue mitochondrial content and distribution induced by the Re-based CORM according to the invention in intact tissues at 24, 48, and 72h after administration. An inactive control (B12-ReAA) was used.

*Mitochondrial biogenesis.* Mitochondrial proliferation was evaluated, qualitatively and quantitatively (using Biorad Image Quant software) by direct fluorescence microscopy of formalin-fixed tissue sections.

*Effects on COHb and MetHb blood levels.* The assessment of carboxyhemoglobin (COHb) and methemoglobin (MetHb) blood levels was performed using co-oximetry (IL-682; Instrumentation Laboratories).

### B12-CORM2 induced mitochondrial biogenesis in multiple tissues

Regardless of route of administration, the use of B12-CORM2 at doses in the range above produced dose-dependent mitochondrial biogenesis in cardiac tissue by 48 to 72 hours. This mitochondrial biogenesis was not limited to the heart, and also included marked mitochondrial proliferation in the lungs, liver, kidneys (medulla and cortex), and brain. Specifically, the *in vivo* cellular distribution of mitochondrial proliferation was broad, and included, but was not limited to cardiomyocytes, skeletal myocytes, hepatocytes, Kupffer cells, lung airway epithelial cells, alveolar type I and alveolar type II epithelial cells, proximal and distal renal tubule cells, blood and tissue macrophages, and Purkinje cells and hippocampal neurons of the brain. Importantly, kidney tubules, which are very sensitive to damage from inflammation, were also intact. No mitochondrial biogenesis was observed following the administration of an inactive control (B12-ReAA), as revealed by photomicrographs.

### Excretion and biological effects

B12-CORM2 was rapidly excreted into the urine of the mouse with peak responses at 30-60 minutes after the injections. The evaluation of the biological effects of B12-CORM2 included the assessment of carboxyhemoglobin (COHb) and methemoglobin (MetHb) blood levels. B12-CORM2, administered to mice by any route of administration at the doses mentioned above, produced increases in COHb of 1 to 3% or no increases in MetHb above 2%. These levels are within the physiological range for both species.

### Example 8: B12-CORM2 stimulates mitochondrial biogenesis and has an anti-fibrotic effect in pulmonary cells and lung tissues, and thereby improves lung function in vivo

*Cell cultures.* Murine lung epithelial alveolar type II cells (MLE-12) and fully differentiated murine type II alveolar epithelial cell line (T7) are grown in Dulbecco's modified Eagle's media (DMEM) containing growth factors and interferon gamma for proliferation at 33 - 35°C and at 39°C in the absence of gamma-interferon cells for terminal differentiation. T7 cells were derived from transgenic H-2Kb-tsA58 mice capable of being passaged as an immortalized cloned cell line in culture. MRC5 human lung fibroblasts are derived from normal fetal lung tissue (ATCC).

*Cell treatments.* Cells were transduced with sh-HO-1 in cells for HO-1 gene silencing: Transfection of HO-1 pRS-shRNA (OriGene, TR504621) performed using FuGENE 6 (Roche), followed by selection on 4 µg/ml puromycin). B12-CORM2 at 25µM was applied over-night. Cells treatments also included exposure to cytomix (LPS, TNF-α and IL-1 b), exposure to TGF-β1, treatment with Bleomycin (once in media at 100 milliunits).

*Mitochondrial and pro-fibrotic readouts.* Analyses included Mitotracker green and NRF-1 stainings, HO-1, SOD2, Akt, CS, Bcl2, TGF-β1, Smad3, NRF1, PGC-1α, cytochrome b, and Tfam mRNA levels by qRT-PCR, and HO-1, PGC-1α, NRF-1, SP-C protein expression measures by Western Blot analyses. The pro-fibrotic state was defined by the extent of Collagen type I and II gene activation and/or collagen expression levels.

*Cell death curves.* The viable cells were counted by Trypan blue exclusion 24 h after exposure to cytomix (LPS, TNF-α and IL-1b) ± B12-CORM2. Mean values ± SE for each group of MLE12 cells were established based on results of at least two independent experiments.

*Fibrin clot sepsis mouse model. S. aureus* (clinical isolate Rosenbach Seattle 1945; ATCC [Manassas, VA] #25923; 1 x 10⁶ to 10⁸ CFU) was prepared, counted, and implanted abdominally in fibrin clots into WT mice under anesthesia. Mice were treated with Vancomycin (3 mg/kg) plus fluid resuscitation (1.0 ml subcutaneously) and/or the same inoculation plus three injections of B12-CORM2 (30 mg/kg/day subcutaneously).

*S. aureus pneumonia model. S. aureus* was instilled directly into the lungs to cause pneumonia, as already described in Athale et al., 2012, Free. Radic. Biol. Med., 53(8), 1584-94. B12-CORM2 (30 mg/kg) was administered subcutaneously once daily for three days. The effect of B12-CORM2 in this model was evaluated using pulmonary inflammatory markers by bronchoalveolar lavage (BAL) fluid analysis for cells, protein and lactate dehydrogenase (LDH), for lung edema (lung wet weight to dry weight (W/D weight ratio)), and for histology using the acute lung injury score (LIS).

*Bleomycin-induced pulmonary fibrosis rodents.* Fibrosis was induced by Bleomycin (500 milliunits) instillation via a cannula inserted into the trachea and down into the lungs, or by subcutaneous injection (1 unit/kg), after anesthesia, in mice and in rats treated or not with B12-CORM2 (3 consecutive subcutaneous injections of 30 mg/kg every 24h) or lung-targeting derivatives. Mice were evaluated daily for body weight and respiratory status. Groups comprised of at least 4 mice per time point. Controls received vehicle alone or vehicle plus an inactive control (B12-ReAA).

*Analyses performed for these in vivo sepsis, pneumonia, and pulmonary fibrosis models.* Lungs were harvested and examined at one or more times after the injuries, including 0, 7, 14 and 28 days. Lung weight was determined and analyses were performed on the controls, affected, and treated lungs. Immunohistologic analyses comprised H&E, collagen content analysis, Masson's trichrome for visualizing fibrotic lesions, fibrosis scores (including the semi-quantitative Ashcroft score *(*Ashcroftet al., 1988, J. Clin. Pathol., 41, 467-70). Total hydroxyproline content of the lung parenchyma was measured by using the method of Woessner (Woessner, 1961, Jr. Arch Biochem. Biophys. 93, 440-7*).* The up-regulation of extracellular matrix (ECM) genes expression, receptor-activated Smads (R-Smads; Smad3/4) nuclear translocation, CTGF expression, myofibroblast proliferation (alpha-smooth muscle actin (αSMA) as a marker), and type I collagen deposition in the interstitium and obliteration of alveolar sacs, type I and III procollagen and fibronectin mRNA expression were also measured. Lung inflammatory alveolar/interstitial edema was analyzed using measurements of lung TNF-α, IL-1β, IL-6, NOS2, IL-18, MCP-1, TGF-β1, and CTGF induced by Bleomycin. Lung cytokine levels were measured in tissue homogenates by ELISA tests. Lung inflammation was also assessed ex vivo by the measurement of bronchoalveolar lavage (BAL) fluid cell count, protein and LDH using standard methods. Vascular leak is also assessed by Evans blue dye extravasation. Protein extraction was performed for pAkt/Akt ratio, HO-1, TGF-β1, Smad3, and fibronectin expression assessment by Western analysis. The Western blots were analyzed by optical densitometry, and the volume density required staining for citrate synthase (CS) and then quantitative analysis by Nikon Element software. The impact on oxidative stress was assessed by the assay of insensitive and non-specific protein carbonyl content, and thioredoxin 1 and 2 and superoxide dismutase 1 and 2 activities in lung tissue. Finally, activation of mitophagy was evaluated by assessing p62, LC3B, and Nix marker (Santa Cruz) protein levels using Western Blot analyses on whole lung homogenates and immunochemistry on 5 micron thick lung sections.

### B12-CORM2 stimulates mitochondrial biogenesis in a HO-1-dependent manner and provides protection from cytokine-induced cell death in murine lung epithelial alveolar type II (MLE12) cells

B12-CORM2-treated MLE12 cells displayed significant increase in mitochondrial biogenesis, revealed by Mitotracker green, as well as by NRF-1 nuclear translocation and activation. Similar effects, although less intense, were observed in MLE-12 cells transduced to overexpress HO-1. Transfection with sh-HO1 blunted B12-CORM2 effects on mitochondrial biogenesis. B12-CORM2 also protected MLE12 cells from cytokine-induced cell death, notably from cytomix-induced cell death (Fig. 6A). MLE-12 cells exposed to an LD50 of LPS, TNF-α, and IL-1β (cytomix) and treated with B12-CORM2 (25 µM) revealed that the pro-survival effect of the Re-based CORM according to the invention was mediated in particular by inducing HO-1 and mitochondrial antioxidant defenses (SOD2 expression), by enhancing the activation of the pro-survival kinase Akt, which is necessary to stimulate the translocation of NRF-1 to the nucleus during mitochondrial biogenesis, by preventing the loss of cytomix-induced citrate synthase (CS), a TCA cycle enzyme that indicates change in mitochondrial mass, and by enhancing the expression of the mitochondrial anti-apoptosis protein Bcl2. These Western blot analyses results are summarized in Table 6, which shows expression levels obtained from three to four independent experiments, normalized to the reference protein β-actin, and expressed in percentage of levels in untreated cells.

**Table 6**

| **Protein expression levels in MLE12 cells (% as compared to untreated cells):** | | | |
|---|---|---|---|
| | **+ B12-CORM2** (SD) | **+ cytomix** (SD) | **+ B12-CORM2 + cytomix** (*SD*) |
| **HO-1** | 180 % (*) (*21.7*) | 211 % (*) (*35.8*) | 326.3 % (*) (*72.1*) |
| **SOD2** | 167.3 % (*) (*17.6*) | 292.7 % (*) (*39.5*) | 232.5 % (*) (*56.6*) |
| **Akt** | 356.7 % (*) (*51.9*) | 279 % (*) (*62.2*) | 482.3 % (**) (*75.4*) |
| **CS** | 147 % (*) (*13.5*) | 108.5% (*9.2*) | 228.8 % (**) (*24.6*) |
| **Bcl2** | 132.5 % (*) (*23.7*) | 93.4 % (*22.7*) | 184.7 % (**) (*33.2*) |
| (*) P<0.05 vs. untreated cells; (**) P<0.05 vs. cytomix alone | | | |

### B12-CORM2 stimulates mitochondrial biogenesis and provides protection by stimulating surfactant protein-C secretion in lung T7 cells

Lung T7 cells (surfactant-secreting cells) were grown in either cell proliferation or in cell differentiation media for 24 hours. The protective effects of B12-CORM2 (25 µM) treatment and induction of mitochondrial biogenesis was investigated by Western Blot analyses. Proteins impacted by B12-CORM2 treatment at 0, 4, and 24h, thus at different levels of maturation, included PGC-1α and NRF-1 (Table 7). The expression of PGC-1α, a major effector of metabolic pathways, was greater during proliferation than during differentiation, indicating an increase in mitochondria to support the energy needed after mitosis. The expression of NRF-1, which also regulates cell cycle pathways, was enhanced in both differentiating and proliferating cells, indicating mitochondrial biogenesis in support of specialized functions in mature cells, such as surfactant production, phagocytosis, or ion pumping. B12-CORM2 also accelerated HO-1 induction in differentiation media (Table 7). B12-CORM2 effect on cell maturation was also demonstrated by the marked increase in surfactant production in differentiation media, as revealed by the acceleration of surfactant protein-C (SP-C) secretion, a critical factor for surfactant film formation and function in reducing surface tension along the alveolar air-liquid interface at end respiration, i.e. a sign of maturation into type II lung cells (Table 7). Positive SP-C and for cytokeratin production was confirmed by immunohistochemical analyses. In other words, B12-CORM2 induced differentiation of T7 cells into SP-C- and cytokeratin-positive alveolar type II epithelial cells.

**Table 7**

| **Protein expression levels in T7 cells (% as compared to untreated cells):** | | | | |
|---|---|---|---|---|
| | **Proliferation media** (*SD*) | | **Differentiation media** (*SD*) | |
| | **4h** | **24 h** | **4h** | **24h** |
| **PGC-1α** | 325 % (*) (*58*) | 1030 % (*) (*128*) | 182 % (*35*) | 370 % (*) (*63*) |
| **NRF-1** | 265 % (*) (*48*) | 542 % (*) (79) | 520 % (*) (*67*) | 765 % (*) (*93*) |
| **HO-1** | 182 % (*35*) | 185 % (*41*) | 165 % (*44*) | 440 % (*) (*75*) |
| **SP-C** | 157 % (*23*) | 208 % (*37*) | 170 % (*) (*25*) | 410 % (*) (*67*) |
| (*) P<0.05 vs. untreated cells (0 h) in corresponding media | | | | |

### B12-CORM2 anti-fibrotic effects and activation of the mitochondrial biogenesis pathway in human lung MRC5 fibroblasts

Since myofibroblasts are one of the key effector cells in pulmonary fibrosis, a progressive life-threatening disease for which no effective therapy exists, and are the primary source of extracellular matrix production (production of excess collagen), the effects of B12-CORM2 on TGF-β-mediated collagen production were assessed in MRC5 human lung myofibroblasts. TGF-β caused changes in fibroblasts phenotype including morphological alterations, expression of Smad proteins, smooth muscle actin, and fibronectin, as well as the production of and of type I and type II collagen.
MRC5 cells were exposed to TGF-β1 to study pro-fibrotic responses including TGF-β1-mediated collagen production. Collagen type I and II gene expression were assessed following B12-CORM2 treatment. B12-CORM2 protective effects were also evaluated against the pro-fibrotic state caused by Bleomycin, a chemotherapeutic drug inducing lung fibrosis. B12-CORM2 markedly inhibited collagen production induced by TGF-β1 (Fig. 6B). Likewise, a single B12-CORM2 treatment blocked collagen mRNA expression (Fig. 6C) and inhibited the activation of TGF-β1 and of the fibrosis marker Smad3 (Fig. 6D and E) in MRC5 cells treated with Bleomycin.
The protective effects of B12-CORM2 in human lung MRC5 fibroblast cells are accompanied by the simultaneous induction of mitochondrial biogenesis. Indeed, B12-CORM2 activates NRF-1 and the mitochondrial biogenesis pathway, even in the presence of Bleomycin (Fig. 6F). Likewise, B12-CORM2-treated (25µM) MRC5 fibroblasts in the presence of TGF-β1, which naturally inhibits the expression of NRF-1 and transcriptional regulators of mitochondrial biogenesis, still highly express NRF-1, PGC-1α, and Tfam (Table 8). B12-CORM2 also induces mitochondrial cytochrome b protein, a surrogate for mitochondrial mass, in the presence of TGF-β1 (Table 8). Furthermore, B12-CORM2 induces increased expression of HO-1 in MRC5 fibroblasts in a dose-dependent manner, already at 6 hours and by 4 fold at 24 hours (Table 9).

**Table 8**

| **Protein expression levels in TGF-β-exposed MRC5 cells treated with B12-CORM2 (% as compared to cells exposed to TGF-β alone)** | | | |
|---|---|---|---|
| | **Day 0** (*SD*) | **Day 1** (*SD*) | **Day 2** (*SD*) |
| **NRF-1** | 103 % (*14*) | 416 % (*) (*46*) | 340 % (**) (*30*) |
| **PGC-1α** | 97 % (*14*) | 306 % (**) (*42*) | 287 % (*) (*30*) |
| **Tfam** | 103 % (9) | 225 % (*) (*32*) | 239 % (**) (*22*) |
| **cytochrome *b*** | 110 % (*11*) | 128 % (*16*) | 239 % (*) (*27*) |
| (*) P<0.05 vs. untreated cells (Day 0); (**) P<0.05 vs. TGF-β alone | | | |

**Table 9**

| **HO-1 mRNA levels in MRC5 cells treated with B12-CORM2 (% as compared to untreated cells)** | | | |
|---|---|---|---|
| | | **B12-CORM2** | |
| | | **10 µM** (*SD*) | **25 µM** |
| **Time of treatment** | **6 h** | 151.6 % (*29*) | 308.9 % (*) (*57*) |
| | **24 h** | 289.5 % (*) (*40*) | 607.7 % (*) (*72*) |
| (*) P<0.05 vs. untreated cells at corresponding time | | | |

### B12-CORM2 stimulates tissue regeneration and repair in lungs in vivo

Following the subcutaneous administration of 100, 500 micrograms or 1mg of B12-CORM2, a marked increase in Sca-1- and Ki67-positive double staining was observed as compared to non-injected mice. This co-staining reveals the stimulated proliferation of resident stem cells in the lung. Furthermore, many alveolar type II cells appeared to be positive for these markers as well. In addition, these same cells were positive at 24 to 48 hours for increased citrate synthase (CS) content and for nuclear NRF-1 staining indicating enhanced mitochondrial biogenesis.
Re-based CORMs of the invention were also tested for their ability to stimulate resident cells in the lung and induce lung tissue repair in three pulmonary lesion rodent models, notably a fibrin clot sepsis model, a *S*. *aureus* pneumonia model, and a Bleomycin instillation model.
In the Bleomycin-introduced pulmonary fibrosis rodent models, lungs harvested 7 days after Bleomycin administration revealed that Bleomycin caused an increase in pulmonary gene expression for TGF-β1, Smad3, and fibronectin, and decreases mitochondrial volume density in the lung parenchyma. B12-CORM2 proved to efficiently block these increases (Fig. 7).
The pro-survival and anti-inflammatory effects of B12CORM2 in the lungs were demonstrated in the fibrin-clot sepsis model. Lungs were harvested and proteins were extracted and analyzed by Western Blot analyses and optical densitometry or by ELISA. All fibrin-clot-inoculated mice were treated with Vancomycin (3 mg/kg) plus fluid resuscitation (1.0 ml subcutaneously). Those who were additionally treated with B12-CORM2 (three subcutaneous injections of 30 mg/kg/day) showed robust and prolonged pro-survival pAkt and HO-1 induction, and by lower TNF-α and higher anti-inflammatory IL-10 levels than vehicle-control mice (Fig. 8 A-D). Furthermore, as mitochondrial autophagy has been shown to be induced by oxidative lung damage and is considered to be a pro-survival response to oxidative stress (Zhang et al., 2013, FASEB J., Jun 14), activation of mitophagy was measured in the lung during sepsis with and without B12-CORM2 treatment. The use of B12-CORM2 during the infection accelerated the induction and allowed the earlier resolution of autophagy in the lung as shown by changes in p62 and LC3B marker protein levels compared with untreated mice (Fig. 8 E-F). These data are supported by immunohistochemical data indicating that mitochondrial autophagy is a major consequence of pneumonia and sepsis in murine lung.

### Repair mediated by B12-CORM2 on diseased human lung tissues

Anti-fibrotic and repair effects of Re-based CORMs according to the invention are tested as follows:
*Disease models.* Cultures of primary epithelial cells and of fibroblasts were collected and isolated from diseased tissue of COPD patients, idiopathic pulmonary fibrosis patients, as well as of patients with non-diseased lungs having undergone partial resection. Cultures were submitted or not to pro-fibrotic stimuli such as TGF-β1, cigarette smoke condition medium, or 100% oxygen over 48 to 72 hours. Cultures were treated with increasing doses of B12-CORM2 or with an inactive control (B12-ReAA).
*Repair and anti-fibrotic assessment.* Impact on type I alveolar cells cell survival and type II cells hyperplasia was tested by direct cell counting. Effects on the extracellular composition was carried out by assessing the deposition of collagen type-I and fibronectin. Epithelial to mesenchymal transition was assessed by lapse time microscopy and immunohistochemistry (expression of E-cadherin and fibronectin). Immunofluorescent detection of αSMA was used as a marker for myofibroblasts differentiation and phospho-Smad3/4 as a marker for the intracellular activity of TGF-βRI and for Smad7 as a marker of endogenous anti-TGF-β activity. Gene expression was also investigated. Finally, impact on kinase activity of TGF-βRI and TGF-βRII were determined using the Promega Kinase-Glo™ kit (Promega, Mannheim, Germany).

### Example 9: B12-CORM2 protective effects in liver

*Cell cultures.* Rat hepatocytes (H411E cells) and mouse hepatoma cells (Hepa 1-6 cells; ATCC, CRL-1830) were grown to near confluence in Dulbecco's modified Eagle's medium (DMEM) and 5% CO2 at 37°C. Mouse primary hepatic stellate cells (D'Ambrosio et al., 2011, PLoS ONE, 6(9), e24993*)* are isolated using a collagenase/pronase perfusion system and differential centrifugation using a modification of the method of D'Ambrosio et al. (D'Ambrosio et al., 2011, PLoS ONE, 6(9), e24993*)*

*B12-CORM2 treatment.* 10, 25 or 50 µM applied overnight (-16 hours) followed by a change to fresh media. An inactive control (B12-ReAA) was used.

*Mitochondrial analyses.* Mitochondrial content was tracked with mtDNA copy number, as well as mRNA and protein levels for citrate synthase (CS) and Tfam, and protein levels of NADH dehydrogenase subunit I and COX subunit I. The redox activation of the mitochondrial biogenesis program was followed using Nrf2 nuclear translocation and changes in HO-1 mRNA and protein levels. The nuclear regulation of the biogeneis program was also tracked using mRNA and protein expression for PGC-1α, NRF-1 and NRF-2 (GABPA). The mitochondrial probes Mitotracker green (Molecular Probes, Invitrogen) and NRF-1 immunocytochemistry were used as described above.

*Fibrosis induction.* mRNA expression levels of specific markers of fibrosis, such as collagen-1a (Col1a1), PPAR gamma, Fibronectin 1 (Fn1), and alpha smooth muscle actin (α-SMA) were measured using real-time RT-PCR.

*Cell treatments:* Confluent cultured cells were exposed to cytomix for 24 hours. B12-CORM2 was added to the medium at the same time as cytomix. Bilirubin, NAC (5mM), and BAY11 (BAY 11-7082, Santa Cruz) were dissolved in PBS and Hemin which was brought up in PBS containing 10% NaOH and then titrated back to pH 7.4. All were applied to the cells once at the start of the experiments.

*Metabolic capacity assessment:* The metabolic state of Hepa 1-6 liver cells was measured using the MTT (3-(4,5-dimethylthiazol-2-yl)-2,5-diphenyltetrazolium bromide, a yellow tetrazole) reduction assay. MTT is reduced to the purple formazan by living cells, which is detected colorimetrically (at 570 nm). Tetrazolium dye assays also measure of loss of cell viability (cytotoxicity) and/or cytostasis induced by medicinal or toxic agents.

*Inflammation assessment.* The mRNA levels for the pro-inflammatory enzyme NOS2 and the anti-inflammatory cytokine interleukin 10 (IL10) were measured by real time RT-PCR.

### B12-CORM2 stimulates mitochondrial biogenesis in rat and mouse hepatocytes in a HO-1-dependent manner

B12-CORM2-treated H411 E cells displayed significant increase in mitochondrial biogenesis, revealed by Mitotracker green, as well as NRF-1 nuclear translocation and activation. Expression levels of HO-1, PGC-1α, N RF-1, Tfam were significantly increased in these cells following treatment with B12-CORM2, in a dose-dependent manner.

### Anti-fibrotic effects of B12-CORM2 on primary hepatic stellate cells

Human hepatic stellate cells (HeSC) are intralobular connective tissue cells with myofibroblast-like features (phenotype) that participate in the maintenance of extracellular matrix homeostasis, and in liver repair, regeneration, and fibrosis. B12-CORM2 (25 µM) was found to induce PPAR gamma, an important factor in the support of metabolism during cell stress that also displays anti-inflammatory effects and promotes the M2 phenotype of macrophages involved in the resolution of inflammation (Table 10). In parallel, B12-CORM2 lead to a reduced expression of Col1a1 (collagen 1a), an extracellular matrix protein involved, when over-produced, in fibrosis (Table 10), and of the alpha smooth muscle actin (α-SMA), a vascular smooth muscle protein and part of the contractile apparatus that serves as a marker of myofibroblast formation (Table 10). Simultaneously, 24h after treatment, B12-CORM2 induces an increase in HO-1 expression levels (Table 10).

**Table 10 (SD: standard deviations)**

| **mRNA expression levels at 24 h in HeSC (% as compared to untreated cells):** | |
|---|---|
| PPARγ | 259 % (*) (*33*) |
| collagen 1α | 55% (*) (*15*) |
| αSMA | 52 % (*) (*12*) |
| HO-1 | 278 % (*) (*23*) |
| (*) P<0.05 vs. untreated cells | |

### B12-CORM2 protective effects against cytomix-induced loss of metabolic capacity and inflammatory response in Hepa 1-6 cells

Cytomix damages and kills liver cells such as Hepa 1-6 cells. The metabolic state of Hepa 1-6 liver cells were measured using the MTT reduction assay. MTT (3-(4,5-dimethylthiazol-2-yl)-2,5-diphenyltetrazolium bromide, a yellow tetrazole) is reduced to the purple formazan by living cells, which is detected colorimetrically. Tetrazolium dye assays also measure of loss of cell viability (cytotoxicity) and/or cytostasis induced by medicinal or toxic agents.
The MTT reduction assay data demonstrates that B12-CORM2 treatment for 24 h, by increasing the rate of MTT reduction, stimulates cell metabolism in control cells and protects against cytomix-induced loss of cell metabolic signal. It therefore shows the pro-metabolic effects and lack of cytotoxicity of the compound of the invention (Fig. 9A). In contrast, exposure to the HO-1 substrate hemin, which was used to induce HO-1 and which causes, when in excess, oxidative stress and cell death, did not cause any significant cell death. Rather, hemin had an additive effect with cytomix, and although hemin is an inducer of HO-1 and endogenous CO production, it did not provide any protection against the cytomix-induced damage. Similarly, the anti-oxidant bilirubin, which is another downstream product of HO-1 (made from biliverdin), had no effect on MTT reduction and therefore on respiratory capacity. In sum, exposure to the HO-1 substrates, hemin or bilirubin, had no effect on the cells and B12-ReAA also has no significant metabolic effect. These observations underline the therapeutic potential of B12-CORM2 as compared to these other compounds whose effects are essentially related to their CO-releasing properties and not to mitochondrial or cytotoxicity.
Furthermore, B12-CORM2 also efficiently reduced the inflammatory response to cytomix, notably by causing a reduction in the expression of pro-inflammatory NOS2 reduction and an increase in anti-inflammatory IL10 expression (Fig. 9B). In contrast, inactive control (B12-ReAA) did not protect cells against cytomix-induced inflammatory response and cell death. Administration of the low molecular weight thiol anti-oxidant N-acetyl cysteine (NAC) was also not protective and, instead, proved to interfere with the anti-inflammatory effect of B12-CORM2, thus supporting the proposed redox mechanism of action of the compound of invention. Finally, the NF-kB inhibitor BAY11 fully blocks the cytomix effect on NOS2 induction and the entire early phase response to cytomix, but only partially reduces B12-CORM2 effects on IL-10, indicating once again an NF-kB-independent anti-inflammatory response through a redox mechanism of action of B12-CORM2.

### Example 10: Effects of B12-CORM2 and kidney-targeting derivatives on renal function and fibrosis

The renal protection potential of Re-based CORMs of the invention is tested on renal function in an acute mouse model as well as on renal lesions and fibrosis in unilateral pedicle clamping or unilateral uretral obstruction models, as follows:

*Animal models.* Mouse and rat models of acute kidney injury (Liet al., 2012, Am. J. Physiol. Renal Physiol., 302, F519-25*).* Following pedicle clamping, the low cortex and external part of the external medulla, i.e. regions with high metabolism and low vascularization features, are greatly affected. Acute renal failure was induced by bilateral clamping. Unilateral pedicle clamping was also performed to assess effects on renal apoptosis, morphology, inflammation, and fibrosis. These unilateral models benefit from an internal negative control, the contralateral non-obstructed kidney. Controls groups consist of sham-operated animals.

*Read-out in acute model.* 24h and 72h post-clamping, the impact of B12-CORM2 or kidney-targeting derivatives on mortality, renal function, creatinine blood levels, renal arterial blood flow velocity, renal tubular injury / necrosis score by immunohistology, renal proinflammatory cytokine and chemokine protein production (including TNF-α, IL-1β, IL-6, IL-10, IFN-γ, Neutrophil gelatinase associated lipocalin (NGAL), monocyte chemoattractant protein-1 (MCP-1)), was assessed as well as percentage of oxyhemoglobin (HbO₂) saturation in kidney.

*Read-out in fibrosis model.* Serum creatinine levels were measured and ut immunohistological analyses on kidney morphology and structure (hematoxylin & eosin, AQP2 (C1-2) and NaPi2a (C3-4) immunostainings), on the development of tubulo-interstitial fibrosis (argentic and Masson trichrome staining, unpolarized and polarized Sirius red staining for quantitative assessment, interstitial fibroblast-specific protein-1 staining, Sircol assay for collagen), on tubular cell apoptosis (TUNEL assay), on tubular cell proliferation (PCNA immunostaining), on peritubular capillary density (isolectin B and VEGF staining), were carried out as well as on interstitial infiltration of monocytes/macrophages (F4/80 staining). Tubular cell proliferation and peritubular capillary density are also examined by Western Blot analyses (measures of PCNA and HIF-1α, respectively). The expression of specific markers of fibrosis (such as collagen-1 and -3, fibronectin, αSMA, TGF-β, phosphorylation of Smad3/4, and HGF), tubular epithelial mass / atrophy (measures of E-cadherin and Na-K-ATPase) was assessed. Oxidative stress was examined by performing 8-OH-2'-dG and 3-nitrotyrosine staining. Changes in the structure and function of podocytes, impact on the renin-angiotensin system, stimulation of connective tissue growth factor (CTGF) and non-enzymatic advanced glycation end-products (AGEs) and receptors for AGEs, which contribute to renal fibrosis, were also examined.

*Mitochondrial biogenesis in all models.* Mitochondrial morphology, and respiration were studied. These analyses were performed on freshly excised livers and kidneys and isolated hepatic and renal mitochondria. Several mitochondrial bioenergetic parameters were tested, including State 3/4 respiration (respiratory control ratio), mitochondrial permeability transition (MTT) and extra-mitochondrial respiration rates. Mitochondrial non-denaturing gel analysis allowed the assessment of the expression levels of the four electron transport complexes (Complexes I-IV), and the ATP synthase (Complex V), cellular and mitochondrial oxidative stress enzymes (MnSOD, Cu/ZnSOD, NOX-4), as well as factors involved in mitochondrial biogenesis (PPARγ coactivator (PGC)-1α, PGC-1β, AMPK, and phospho-AMPK). Mitochondrial H₂O₂ emission was measured by monitoring Amplex Red oxidation. Mitochondrial ROS production was measured using MitoSOX™ Red reagent.

## Claims

1. A Re-based CORM, a derivative or a mixture thereof for use in the treatment or prevention of fibrosis or fibrotic lesions or in the treatment or prevention of systemic inflammatory response syndrome or sepsis or in the promotion of tissue growth and/or repair and/or improving cell/tissue survival.

2. A Re-based CORM, a derivative or a mixture thereof for use according to claim 1 wherein fibrosis or fibrotic lesions are fibrosis or fibrotic lesions from an organ selected from heart, lung, kidney and liver.

3. A Re-based CORM, a derivative or a mixture thereof for use according to claim 1, wherein the tissue is a tissue selected from a heart tissue, a lung tissue, a kidney tissue and a liver tissue.

4. A Re-based CORM for use according to any one of claims 1 to 3, wherein the Re-based CORM is of is of the following formula (I): 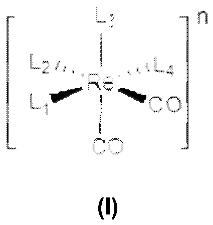 wherein Re is Re(I), Re(II) or Re(III), preferably Re(II); n is selected from 3- to 3+, such as 2-, 1-, 0, 1+ and 2+, in particular 1-, 0, 1+; L1, L2, L3 and L4 denote in each case independently of one another pharmaceutically acceptable monodentate, bidentate, tridentate ligands or combinations thereof.

5. A Re-based CORM for use according to claim 4, wherein L1, L2, L3 or L4 is Cyanocobalamin.

6. A Re-based CORM for use according to any one of claims 1 to 5, wherein the compound of Formula (I) is of the following formula:

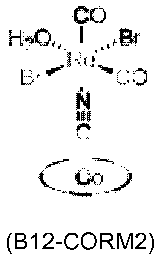

7. A Re-based CORM for use according to any one of claims 3 to 6, wherein the Re-based CORM, derivative thereof or mixture thereof is to be administered *ex-vivo* in said tissue.

8. A Re-based CORM for use according to any one of claims 1 to 7, wherein said Re-based CORM, a derivative thereof or a mixture thereof is to be co-administered with cells selected from stem cells, progenitor cells, precursor cells, totipotent cells, pluripotent cells, multipotent cells or oligopotent cells.

9. A composition comprising a Re-based CORM, a derivative thereof or a mixture thereof combined with at least one co-agent useful in the promotion of tissue growth, repair or survival or useful in the improvement of cell survival, or useful in the treatment or prevention of fibrosis or fibrotic lesions.

10. A composition according to claim 9 wherein the composition is a composition for preservation or preparation of cells, organs or tissues.

11. A composition according to claim 9 wherein the composition is a pharmaceutical composition and further comprises a pharmaceutically acceptable carrier, diluent or excipient.

12. An *ex-vivo* method of preventing tissue fibrotic lesion formation and inducing tissue regeneration comprising a step of contacting said tissue with a Re-based CORM, a derivative thereof or a mixture thereof.

13. A method of preparation of a cell composition for cell-based therapy comprising a step of contacting a cell with a Re-based CORM, a derivative thereof or a mixture thereof, wherein said cell is selected from a stem, a progenitor, a precursor, a totipotent, a pluripotent, a multipotent and an oligopotent cell.

14. A preservation or preparation medium for cells, organs or tissues comprising a Re-based CORM, a derivative thereof or a mixture thereof.

15. A method of inducing maturation of a stem, or a progenitor, or a precursor, or a totipotent, or a pluripotent, or a multipotent or an oligopotent cell, comprising inducing expression of heme oxygenase 1 (HO-1) in said cell.

16. A method according to claim 15 comprising a step of contacting said cell with a Re-based CORM, a derivative thereof or a mixture thereof.
